# EUROPEAN PATENT APPLICATION

(11) **EP 4 847 633 A2**
(43) Date of publication of application: **02.12.2026**
(21) Application number: 26205411.7
(22) Date of filing: 06.04.2023
(51) Int. Cl.: C12Q 1/6895

(54) **PLANT REGULATORY ELEMENTS AND USES THEREOF FOR AUTOEXCISION**

(30) Priority: 12.04.2022 US 202263329964 P
(62) Document number(s) of the earlier application(s) in accordance with Art. 76 EPC: 23789076.9 / 4 508 221
(71) Applicant: Monsanto Technology LLC, St. Louis, MO 63167 (US)
(72) Inventor: Flasinski, Stanislaw, St. Louis (US); Marengo, Matthew S., St. Louis (US); Ye, Xudong, St. Louis (US)
(74) Representative: BIP Patents

(57) **Abstract**

Recombinant DNA molecules and constructs are provided that are useful for modulating gene expression in plants. One or more expression cassette(s) of a recombinant DNA molecule or construct may be excised from transgenic plants following transformation by the presence of flanking site-specific recombination sites in the recombinant DNA molecule or construct by expression of a recombinase enzyme encoded by the recombinant DNA molecule or construct. Such a recombinase system may be used to remove such expression cassette(s) from plants transformed with the recombinant DNA construct or vector. The recombinase transgene may be operably linked to a promoter for autoexcision in transformed plants without crossing to a different transgenic line expressing the recombinase. Methods for causing autoexcision of one or more expression cassette(s) in a transgenic plant, and plants and cells containing or transformed with a recombinant DNA molecule or construct of the present disclosure, are also provided.

## Description

### REFERENCE TO RELATED APPLICATIONS

This application claims the benefit of United States provisional application No. 63/329,964, filed April 12, 2022, herein incorporated by reference in its entirety.

### INCORPORATION OF SEQUENCE LISTING

The sequence listing that is contained in the file named "MONS556WO_ST26.xml", is 51 bytes (as measured in Microsoft Windows^{®}), was created on March 28, 2023, and is filed herewith by electronic submission and incorporated by reference herein.

### FIELD OF THE INVENTION

The invention relates to the field of plant molecular biology and plant genetic engineering. More specifically, the invention relates to DNA molecules useful for modulating site-specific recombinase gene expression in plants.

### BACKGROUND

Regulatory elements are genetic elements that regulate gene activity by modulating the transcription of an operably linked transcribable DNA sequence. Such elements may include promoters, leaders, introns, and 3´ untranslated regions and are useful in the field of plant molecular biology and plant genetic engineering.

The use of transgenic technology has provided many beneficial traits for agricultural purposes but has encountered several challenges. One concern is related to the presence of marker genes conferring antibiotic or herbicide resistance in the transgenic crop plants. In addition, there may be other transgene cassettes or DNA sequences that are designed for a particular purpose and present in the initial transformation but are not needed in the final transgenic product. Removal of such marker genes and the other unwanted expression cassettes and DNA sequences is highly desirable in the field of plant biotechnology.

A number of strategies have been designed for the generation of marker-free transgenic plants. For example, removal of the marker gene expression cassette can be done using a two T-DNA transformation system or a site-specific recombinase system.

The two T-DNA transformation system utilizes a binary plant transformation vector that comprises two separate T-DNAs (Two T-DNA transformation system). One T-DNA comprises the marker gene expression cassette. The other T-DNA comprises the expression cassette(s) for the gene(s) of interest that are intended to remain in the transgenic plant. The plant cell can be transformed through *Agrobacterium*-mediated transformation. Each T-DNA can be integrated into separate chromosomes of the transformed plant cell genome. After transformation and plant regeneration, the R₀ plants are self-crossed, resulting in R₁ progeny. R₁ progeny plants are selected that have the T-DNA comprising the expression cassette(s) intended for the final transgenic product but lack the T-DNA comprising the marker gene expression cassette(s) (see, e.g., Komari, T. et al., (1996) Vectors carrying two separate T-DNAs for co-transformation of higher plants mediated by Agrobacterium tumefaciens and segregation of transformants free from selection markers, The Plant Journal, 10(1):165-174). The two T-DNA transformation system has some drawbacks with respect to efficiency. In the two T-DNA transformation system, transformant R₀ plants can have more than one copy of either or both T-DNAs that may have to be excluded, and the percentage of plants passing selection that possess only one copy of each T-DNA can be low.

Another system to remove marker gene expression cassettes from the transgenic plant relies on excision through use of a site-specific recombinase. A number of site-specific recombinases can be used, such as Cre-recombinase, Flp-recombinase (Lyznik, L. et al., (2000) Gene Transfer Mediated by Site-Specific Recombination Systems, Plant Molecular Biology Manual N1, 1-26), R-recombinase (Machida, C. et al., (2000) Use of the R-RS Site-Specific Recombination System in Plants, Plant Molecular Biology Manual N2, 1-23), or Gin-Recombinase (Maeser, S. et al., (1991). The Gin recombinase of phage Mu can catalyze site-specific recombination in plant protoplasts, Mol Gen Genet, 230: 170-176). Essentially, within the construct, such as a T-DNA insertion, the marker gene expression cassette(s) are flanked by site-specific recombinase recognition sequences, such that the construct sequence between the site-specific recombinase recognition sequences can be excised by expression of the recombinase. Expression cassette(s) that are intended to remain in the transgenic plant after excision are present in the construct outside of the site-specific recombinase recognition sequences of the construct.

Removal of the expression cassettes flanked by the site-specific recombinase recognition sequences can be accomplished using a crossing strategy or through autoexcision. In a crossing strategy, plants (e.g., R1 progeny) preferably homozygous for the presence of the construct are crossed with another line of transgenic plants transformed with an expression cassette used for the expression of the site-specific recombinase. The resulting F₁ progeny are then selected for the presence of the construct which has had the expression cassettes flanked by the site-specific recombinase recognition sequences excised. In the case of autoexcision, an additional expression cassette encoding a site-specific recombinase is present in the construct with the other expression cassette(s) to be excised between or flanked by the site-specific recombinase recognition sequences, such that all such expression cassettes are excised by the site-specific recombinase. Often a promoter will have a preference or specificity for driving expression in a specific type of cell or tissue. Not all promoters and expression elements are suitable for efficient autoexcision, and much experimentation is needed to identify the right promoter to drive recombinase expression along with additional expression elements, such as introns and 3´ UTRs, that modulate the recombinase expression to achieve the desired excision frequency and outcome.

There is a need for expression elements that drive efficient autoexcision in a crop plant(s). The present disclosure provides several expression elements identified through many years of experimentation that can be used to drive expression of a recombinase and produce efficient autoexcision of the marker and/or recombinase transgenes and possibly other expression cassette(s) in a number of crop species following transformation.

### SUMMARY

The invention provides gene regulatory elements for use in plants to drive a site-specific recombinase that will result in efficient autoexcision of marker gene expression cassettes as well as expression cassettes used in genome editing. The invention also provides recombinant DNA molecule constructs comprising the regulatory elements. The present invention also provides constructs comprising the regulatory elements. In one embodiment, the regulatory elements are operably linked to a site-specific recombinase. In other embodiments, the regulatory elements are comprised within constructs comprising at least three transgene cassettes. The present invention also provides methods of using the regulatory elements and making and using the recombinant DNA molecules and constructs comprising the regulatory elements. The present invention also provides a synthetic Cre-recombinase coding sequence for expression in a plant cell.

Thus, in one aspect, the invention provides a recombinant DNA construct comprising a DNA regulatory sequence selected from the group consisting of: (a) a sequence with at least about 80 percent sequence identity to any of SEQ ID NOs:1-15; (b) a sequence comprising any of SEQ ID NOs:1-15; and (c) a fragment of (i) any of SEQ ID NOs:1-15 or (ii) any sequence with at least 80 percent sequence identity to any of SEQ ID NOs:1-15, wherein the fragment has gene-regulatory activity; wherein the sequence is operably linked to a heterologous transcribable DNA sequence encoding a site-specific recombinase. In specific embodiments, the recombinant DNA construct comprises a DNA regulatory sequence having at least about 80 percent, at least about 81 percent, at least about 82 percent, at least about 83 percent, at least about 84 percent, at least about 85 percent, at least about 86 percent, at least about 87 percent, at least about 88 percent, at least about 89 percent, at least about 90 percent, at least 91 percent, at least 92 percent, at least 93 percent, at least 94 percent, at least 95 percent, at least 96 percent, at least 97 percent, at least 98 percent, or at least 99 percent sequence identity to the DNA sequence of any of SEQ ID NOs:1-15. In particular embodiments, the DNA regulatory sequence comprises a regulatory element with gene regulatory activity. In some embodiments, the regulatory element comprises a promoter. In still other embodiments, the regulatory element comprises an intron. In still other embodiments, the regulatory element comprises a 3´ UTR. In still other embodiments, the heterologous transcribable DNA sequence comprises a gene encoding a site-specific recombinase. In other embodiments, the site-specific recombinase is selected from the group consisting of a Cre-recombinase, an Flp-recombinase, an R-recombinase, and a Gin-recombinase. In yet another embodiment, the site-specific recombinase is a Cre-recombinase.

In another aspect, the recombinant DNA construct further comprises one or both of the following expression cassettes: a selectable marker transgene; and/or a transgene of agronomic interest. In another embodiment, the recombinant DNA construct further comprises a pair of site-specific recombination site sequences flanking one or both of the transcribable DNA sequences encoding the site-specific recombinase and/or the selectable marker transgene, wherein the site-specific recombination sites can be cleaved by the site-specific recombinase. In further embodiments the selectable marker transgene of the recombinant DNA construct confers resistance to an herbicide or antibiotic. In other embodiments, the site-specific recombination site sequences of the recombinant DNA construct are each selected from the group consisting of LoxP, FRT, RS, and GIX. In specific embodiments the site-specific recombination site sequences of the recombinant DNA construct are each a LoxP site. In other embodiments, the site-specific recombination site sequence of the recombinant DNA construct each comprise SEQ ID NO:20.

In another aspect, the transgene of agronomic interest of the recombinant DNA construct confers herbicide tolerance in plants. In some embodiments, the transgene of agronomic interest of the recombinant DNA construct confers pest or disease resistance in plants. In further embodiments, the transgene of agronomic interest of the recombinant DNA construct confers increased yield or stress tolerance in plants. In yet other embodiments, the transgene of agronomic interest of the recombinant DNA construct encodes a dsRNA, a miRNA, or an siRNA.

In another aspect, the recombinant DNA construct further comprises one or both of the following: an expression cassette encoding a guide RNA; and/or an expression cassette encoding a site-specific nuclease. The recombinant DNA construct also comprises site-specific recombination site sequences flanking one or more of the transcribable DNA sequence encoding the site-specific recombinase, the selectable marker transgene, the expression cassette encoding the guide RNA, and/or the expression cassette encoding the site-specific nuclease, wherein the site-specific recombination sites can be cleaved by the site-specific recombinase. In further embodiments the guide RNA comprises a targeting sequence that targets a sequence in the genome of a eukaryotic cell for genome editing or site-specific integration. In another embodiment, the eukaryotic cell is a plant cell. In yet another embodiment, the recombinant DNA construct comprises two or more expression cassettes encoding two or more guide RNAs. In a further embodiment, the recombinant DNA construct comprises two, three, four, five, six, seven, eight, nine, or ten different expression cassettes encoding guide RNAs. In further embodiments, the site-specific nuclease is a RNA-guided endonuclease or CRISPR associated nuclease. In another embodiment, the RNA-guided endonuclease or CRISPR associated nuclease is selected from the group consisting of Cas1, Cas1B, Cas2, Cas3, Cas4, Cas5, Cas6, Cas7, Cas8, Cas9, Cas10, Cas12a, Cys1, Cys2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, CasX, and CasY. In specific embodiments, the RNA-guided endonuclease or CRISPR associated nuclease is Cas12a or Cas9.

In another aspect, provided herein is a DNA molecule or vector comprising the recombinant DNA construct. In another embodiment, a DNA transformation vector comprises the recombinant DNA construct and a T-DNA segment bounded by a left border and a right border. In further embodiments, the transcribable DNA sequence encoding the site-specific recombinase is located between the left border and the right border of the T-DNA segment within the DNA transformation vector. In yet another embodiment, the DNA transformation vector comprises the recombinant DNA construct and a T-DNA segment with left border and a right border, wherein one or more of the transcribable DNA sequence encoding the site-specific recombinase, the selectable marker transgene and/or the transgene of agronomic interest is/are located between the left border and the right border of the T-DNA segment. In further embodiments, the DNA transformation vector comprises the recombinant DNA construct and a T-DNA segment with left border and a right border, wherein one or more of the transcribable DNA sequence encoding the site-specific recombinase, the selectable marker transgene, the transgene of agronomic interest, the expression cassette encoding the guide RNA and/or the expression cassette encoding the site-specific nuclease is/are located between the left border and the right border of the T-DNA segment.

In another aspect, provided herein is a transgenic plant, plant part, or plant cell comprising the recombinant DNA construct. The recombinant DNA construct is stably transformed into the genome of the transgenic plant, plant part or plant cell. The transgenic plant, plant part or plant cell is a corn, soybean, cotton or canola plant, plant part or plant cell. Also provided herein, is a bacterial cell comprising the recombinant DNA construct or the transformation vector.

In another aspect, provided herein is a method for producing a transgenic plant or plant part, comprising (a) transforming a plant cell of an explant with a DNA molecule or vector comprising the recombinant DNA construct to produce one or more transformed plant cells comprising the recombinant DNA construct stably transformed into the genome of the one or more transformed plant cells; (b) regenerating or developing a transgenic plant from the explant, wherein the transgenic plant comprises the recombinant DNA construct stably transformed into the genome of one or more cells of the transgenic plant. In one embodiment, the plant cell is transformed via *Agrobacterium*-mediated transformation or *Rhizobium*-mediated transformation. In another embodiment, the plant cell is transformed via microprojectile-mediated transformation or particle bombardment-mediated transformation. In yet another embodiment, the transgenic plant and plant cell are a corn, soybean, cotton or canola plant and plant cell, respectively. In yet another embodiment, the method further comprises: (c) separating or harvesting a plant part from the transgenic plant.

In another aspect, provided herein is a method for excising an expression cassette from the genome of a transgenic plant, comprising: (a) transforming a plant cell with a DNA molecule or vector comprising the recombinant DNA construct of any of embodiments 8-25 to produce one or more transformed plant cells comprising the recombinant DNA construct stably transformed into the genome of the one or more transformed plant cells; (b) regenerating or developing a transgenic plant at least in part from the one or more stably transformed plant cells; (c) crossing the transgenic plant to itself or another plant; and (d) selecting one or more progeny plants in which one or both of the transcribable DNA sequence encoding the site-specific recombinase and/or the selectable marker transgene between the pair of site-specific recombination site sequences of the recombinant DNA construct are excised and no longer present in the genome of the progeny plants. In further embodiments of the method, the recombinant DNA construct further comprises one or both of the following expression cassettes between the pair of site-specific recombination site sequences of the recombinant DNA construct: an expression cassette encoding a guide RNA and/or an expression cassette encoding a site-specific nuclease, and wherein one or more progeny plants are selected in which one or more of the transcribable DNA sequence encoding the site-specific recombinase, the selectable marker transgene, the expression cassette encoding the guide RNA, and/or the expression cassette encoding the site-specific nuclease of the recombinant DNA construct are excised and no longer present in the genome of the progeny plants. In specific embodiments, the transgenic plant and plant cell are a corn, soybean, cotton or canola plant and plant cell, respectively. In another embodiment the method further comprises (e) separating or harvesting a plant part from one or more of the progeny plants. In yet another embodiment, the method further comprises (f) crossing one or more of the progeny plants to itself or another plant.

In another aspect, the invention provides a recombinant DNA construct comprising a DNA sequence selected from the group consisting of: (a) a sequence with at least about 85 percent sequence identity to any of SEQ ID NOs:8, 10, 11, 12, and 14; (b) a sequence comprising any of SEQ ID NOs:8, 10, 11, 12, and 14; and (c) a fragment of any of SEQ ID NOs:8, 10, 11, 12, and 14, wherein the fragment has gene-regulatory activity; wherein the sequence is operably linked to a heterologous transcribable DNA molecule. In specific embodiments, the recombinant DNA construct comprises a DNA sequence having at least about 85 percent, at least about 86 percent, at least about 87 percent, at least about 88 percent, at least about 89 percent, at least about 90 percent, at least 91 percent, at least 92 percent, at least 93 percent, at least 94 percent, at least 95 percent, at least 96 percent, at least 97 percent, at least 98 percent, or at least 99 percent sequence identity to the DNA sequence of any of SEQ ID NOs:8, 10, 11, 12, and 14.

In another aspect, provided herein are transgenic plant cells comprising a recombinant DNA construct comprising a DNA sequence selected from the group consisting of: (a) a sequence with at least about 85 percent sequence identity to any of SEQ ID NOs:8, 10, 11, 12, and 14; (b) a sequence comprising any of SEQ ID NOs:8, 10, 11, 12, and 14; and (c) a fragment of any of SEQ ID NOs:8, 10, 11, 12, and 14, wherein the fragment has gene-regulatory activity; wherein the DNA sequence is operably linked to a heterologous transcribable DNA molecule. In certain embodiments, the transgenic plant cell is a monocotyledonous plant cell. In other embodiments, the transgenic plant cell is a dicotyledonous plant cell.

In still yet another aspect, further provided herein is a transgenic plant, or part thereof, comprising a recombinant DNA construct comprising a DNA sequence selected from the group consisting of: a) a sequence with at least 85 percent sequence identity to any of SEQ ID NOs:8, 10, 11, 12, and 14; b) a sequence comprising any of SEQ ID NOs:8, 10, 11, 12, and 14; and c) a fragment of any of SEQ ID NOs:8, 10, 11, 12, and 14, wherein the fragment has gene-regulatory activity; wherein the sequence is operably linked to a heterologous transcribable DNA molecule. In specific embodiments, the transgenic plant is a progeny plant of any generation that comprises the recombinant DNA molecule. A transgenic seed comprising the recombinant DNA molecule that produces such a transgenic plant when grown is also provided.

In another aspect, the invention provides a method of producing a commodity product comprising obtaining a transgenic plant or part thereof containing a recombinant DNA construct of the invention and producing the commodity product therefrom. In one embodiment, the commodity product is seeds, processed seeds, protein concentrate, protein isolate, starch, grains, plant parts, seed oil, biomass, flour and meal.

In still yet another aspect, the invention provides a method of producing a transgenic plant comprising a recombinant DNA construct of the invention comprising transforming a plant cell with the recombinant DNA construct of the invention to produce a transformed plant cell and regenerating a transgenic plant from the transformed plant cell.

In another aspect, provided herein is a synthetic Cre-recombinase coding sequence designed for expression in a plant cell having at least about 90 percent, at least 91 percent, at least 92 percent, at least 93 percent, at least 94 percent, at least 95 percent, at least 96 percent, at least 97 percent, at least 98 percent, or at least 99 percent sequence identity to the DNA sequence of SEQ ID NO:17.

### BRIEF DESCRIPTION OF THE SEQUENCES

**SEQ ID NO:1** is a DNA sequence of a regulatory expression element group (EXP), EXP-Zm.BabyBoom2 comprising a promoter (P-Zm.BabyBoom2:2) operably linked 5´ to a leader (L-Zm.BabyBoom2:1), operably linked 5´ to an intron (I-Zm.BabyBoom2:1).
**SEQ ID NO:2** is a DNA sequence of a promoter, P-Zm.BabyBoom2:2.
**SEQ ID NO:3** is a DNA sequence of a leader, L-Zm.BabyBoom2:1.
**SEQ ID NO:4** is a DNA sequence of an intron, I-Zm.BabyBoom2:1.
**SEQ ID NO:5** is a DNA sequence of a 3´ UTR, T-Zm.BabyBoom2:1.
**SEQ ID NO:6** is a DNA sequence of an EXP, EXP-Zm.GRMZM2G512113_GSI85 comprising a promoter and leader (P-Zm.GRMZM2G512113:1) operably linked 5´ to an intron, I-Zm.GSI85.nno:1.
**SEQ ID NO:7** is a DNA sequence of a promoter and leader, P-Zm.GRMZM2G512113:1.
**SEQ ID NO:8** is a DNA sequence of a synthetic intron, I-Zm.GSI85.nno:1.
**SEQ ID NO:9** is a DNA sequence of a 3´ UTR, T-Zm.GRMZM2G512113:1.
**SEQ ID NO:10** is DNA sequence of an EXP, EXP-SETvi.SPO11-1_Eef7 comprising a promoter (P-SETvi.SPO11-1:1) operably linked 5´ to a leader (L-SETvi.SPO11-1:1), operably linked 5´ to an intron (I-SETit.Eef7:2).
**SEQ ID NO:11** is a DNA sequence of a promoter, P-SETvi.SPO11-1:1.
**SEQ ID NO:12** is a DNA sequence of a leader, L-SETvi.SPO11-1:1.
**SEQ ID NO:13** is a DNA sequence of an intron, I-SETit.Eef7:2.
**SEQ ID NO:14** is a DNA sequence of a 3´ UTR, T-SETvi.SPO11-1:1.
**SEQ ID NO:15** is a DNA sequence of a 3´ UTR, T-SACra.Hsp16.9:29.
**SEQ ID NO:16** is a DNA sequence of a coding sequence encoding Cre-recombinase (GOI-Cre_1) comprising a processable intron used for expression in a plant cell.
**SEQ ID NO:17** is a DNA sequence of a synthetic coding sequence encoding Cre-recombinase (GOI-Cre_2) comprising a processable intron used for expression in a plant cell.
**SEQ ID NO:18** is a DNA sequence of a coding sequence encoding β-glucuronidase (GUS) comprising a processable intron.
**SEQ ID NO:19** is a DNA sequence of a synthetic coding sequence encoding the insect toxin molecule TIC10746_3 used for expression in a plant cell.
**SEQ ID NO:20** is a DNA sequence of a Cre-recombinase recognition sequence, LoxP.
**SEQ ID NO:21** is a DNA sequence of a synthetic coding sequence encoding the selectable marker, GOI-CP4-EPSPS used for selection of transformed plant cells using glyphosate selection.

### DETAILED DESCRIPTION

The invention provides gene regulatory elements for use in plants to drive expression of a site-specific recombinase that will result in efficient autoexcision of marker gene expression cassettes. The invention also provides constructs and recombinant DNA molecules comprising the regulatory elements. The invention also provides methods for autoexcising at least two transgene expression cassettes from the genome of a transgenic plant through the use of a construct comprising a transgene cassette wherein the gene regulatory elements described herein are operably linked to a site-specific recombinase gene.

The following definitions are provided for certain terms and phrases used herein. Unless otherwise defined in the present disclosure, terms and phrases used herein are to be understood according to their conventional meaning by those skilled and knowledgeable in the relevant art.

### Site-specific Recombinases and Excision of a DNA Segment

As used herein, a "site-specific recombinase" is an enzyme that binds to specific DNA recognition sequences and catalyzes the cleavage of DNA, DNA strand exchange, and the rejoining of the DNA between two site-specific recombinase site sequences. "Site-specific recombination," or "site-specific recombinase system," or "site-specific recombinase technologies," or "site-directed recombination," or "site-directed recombinase system," or "site-directed recombinase technologies," describes a variety of specialized recombination processes that involve reciprocal exchange between defined DNA sites. As used herein, the term "flanking" refers to two or more sequences, such as site-specific recombination site sequence(s), that are located on either side of one or more specific locus/loci, gene(s), sequence(s), transgene(s), or expression cassette(s). The site-specific recombination site sequences may be cloned within a recombinant DNA construct 5´ and 3´ relative to a segment of DNA (i.e., flanking the segment of DNA) comprising the expression cassettes under which recombination will occur. Depending on the initial arrangement of the parental site-specific recombination sites, site-specific recombination has one of three possible outcomes: integration (insertion of a foreign DNA segment), excision (removal of a DNA segment), or inversion (rotation of a DNA segment 180 degrees before rejoining the two end fragments). Integration results from recombination between sites on separate DNA molecules (provided that at least one of the parental chromosomes is circular) and occurs with a uniquely defined orientation.

For recombination sites located on the same DNA molecule or chromosome, the outcome can be determined by their relative orientation. While inversion of a DNA segment can result from exchange between inverted (head-to-head) sites, excision can result from recombination between sites in a head-to-tail orientation (Nigel et al. (2006) Mechanisms of Site-Specific Recombination. Annu. Rev. Biochem, 75: 567-605). A number of site-specific recombinases can be used for excision of DNA between two site-specific recombinase recognition sites, such as Cre-recombinase which recognizes Lox sites, Flp-recombinase which recognizes FRT sites (see, e.g., Lyznik, L. et al., (2000) Gene Transfer Mediated by Site-Specific Recombination Systems, Plant Molecular Biology Manual N1, 1-26), R-recombinase which recognizes RS sites (see, e.g., Machida, C. et al., (2000) Use of the R-RS Site-Specific Recombination System in Plants, Plant Molecular Biology Manual N2, 1-23), or Gin-Recombinase which recognizes GIX sites (see, e.g., Maeser, S. et al., (1991) The Gin recombinase of phage Mu can catalyze site-specific recombination in plant protoplasts, Mol Gen Genet, 230: 170-176). Each of the above site-specific recombinase systems have been shown to work in plants. The Cre/Lox site-specific recombinase system is the most frequently relied upon system for marker excision in plant biotechnology.

Site-specific recombinases can be used in plant biotechnology to remove marker gene expression cassettes as well as other expression cassettes and DNA segments from a transgenic plant. Typically, a plant is transformed with a recombinant DNA construct or vector that comprises multiple expression cassettes. The expression cassettes can be used to express transgenes that provide favorable characteristics to the plant as well as transgenes used as markers to select for the transformed plant cells such as antibiotic resistant genes, herbicide tolerant genes, or other transgenes useful in the selection process. The transgene cassettes for the marker genes are flanked by a pair of site-specific recombinase recognition sites. After transformation and selection, the regenerated transformed plants are grown. Excision of the marker genes can then be removed through various crossing strategies, either through crossing with a site-specific recombinase expressing line of plants or through autoexcision.

Crossing using a site-specific recombinase expressing line of plants is often carried out as follows. The R₀ transformed plants are allowed to self-cross. R₁ progeny plants are then selected for the presence of the recombinant DNA construct. The selected R₁ progeny plants are then allowed to self-cross, and R₂ progeny plants are selected that are homozygous for the recombinant DNA construct insertion. The homozygous R₂ progeny plants are then crossed with another line that expresses a recombinase. As a result of this cross, the recombinase excises the marker gene expression cassette(s) that are flanked by the site-specific recombinase recognition sequences, resulting in F₁ progeny plants that comprise the desired expression cassette(s) but with the marker gene expression cassette(s) excised out of the genome. The resulting F₁ progeny are then allowed to self-cross, and F₂ progeny plants are selected that lack the recombinase but are homozygous for the now modified recombinant DNA construct insertion.

Another strategy to remove the marker gene expression cassette(s) is through autoexcision. Similar to the excision approach above, an expressed recombinase is used to excise the marker gene expression cassette(s), but instead of crossing the transformed plants with another line that expresses the recombinase, a recombinase gene expression cassette is located within the same recombinant DNA construct and is flanked by the site-specific recombinase site sequences along with the marker gene expression cassette(s). Expression cassette(s) that are intended to remain in the transgenic plant after autoexcision are present in the recombinant DNA construct outside of the site-specific recombinase site sequences. After transformation and plant regeneration, the R₀ plants containing the recombinant DNA construct are generated. Those R₀ plants can then be self-crossed, and the resulting R₁ progeny plants can be selected for the presence of the altered recombinant DNA construct in which the marker gene expression cassette(s) and recombinase expression cassette have been excised. The advantage of an autoexcision system is that one can remove the marker gene expression cassette(s) in fewer generations than when a site-specific recombinase excision system is used that requires crossing with another line that expresses the site-specific recombinase.

A complicating factor for autoexcision is to find expression elements that provide expression of the site-specific recombinase at the right time and at the right tissues for autoexcision to produce marker-free R₁ progeny plants. Not all expression elements will provide a successful outcome for autoexcision to efficiently occur. In addition, an expression element may only provide efficient autoexcision in a particular crop species such as corn, soybean, or cotton, but not all three. Therefore, much experimentation has been done to identify the promoters of the present invention.

### DNA Molecules

As used herein, the term "DNA" or "DNA molecule" refers to a double-stranded DNA molecule of genomic or synthetic origin, *i.e.*, a polymer of deoxyribonucleotide bases or a DNA molecule. As used herein, the term "DNA sequence" refers to the nucleotide sequence of a DNA molecule, read from the 5´ (upstream) end to the 3´ (downstream) end.

As used herein, a "recombinant DNA molecule" or "recombinant DNA construct" is a DNA molecule or construct, respectively, comprising a combination of DNA sequences that would not naturally occur together without human intervention. For instance, a recombinant DNA molecule may comprise at least two DNA sequences heterologous with respect to each other, a DNA sequence that deviates from DNA sequences that exist in nature, a synthetic DNA sequence, and/or a DNA sequence that has been incorporated into a host cell's genomic DNA by genetic transformation, genome editing, or site-specific integration.

As used herein, a "synthetic nucleotide sequence" or "artificial nucleotide sequence" or "synthetic coding sequence" is a nucleotide sequence that is not known to occur in nature or that is not naturally occurring. Preferably, synthetic nucleotide sequences share little or no extended homology to natural sequences. Extended homology in this context generally refers to 100% sequence identity extending beyond about 25 nucleotides of contiguous sequence. A synthetic gene regulatory element of the present invention is the synthetic intron, I-Zm.GSI85.nno:1 (SEQ ID NO:8). A synthetic coding sequence for example, is the Cre-recombinase coding sequence, GOI-Cre_2 presented as SEQ ID NO:17.

Reference in this application to an "isolated DNA molecule", or an equivalent term or phrase, is intended to mean that the DNA molecule is one that is present alone or in combination with other compositions, but not within its natural environment. For example, nucleic acid elements such as a coding sequence, intron sequence, untranslated sequence, leader sequence, promoter sequence, transcriptional termination sequence, and the like, that are naturally found within the genome of an organism are not considered to be "isolated" so long as the element is native to the genome of the organism and at the location within the genome in which it is naturally found. However, each of these elements, and subparts of these elements, would be "isolated" within the scope of this disclosure so long as the element is not within its native genome and/or present at a location within the genome where it is naturally found. For the purposes of this disclosure, any transgenic nucleotide sequence, *i.e*., the nucleotide sequence of the DNA inserted into the genome of cells of a plant or bacterium, or present in an extrachromosomal vector, would be considered to be an isolated nucleotide sequence whether it is present within the plasmid or similar vector used to transform cells, within the genome of the plant or bacterium, or in detectable amounts in tissues, progeny, biological samples or commodity products derived from the plant or bacterium.

As used herein, the term "sequence identity" refers to the extent to which two optimally aligned polynucleotide sequences or two optimally aligned polypeptide sequences are identical. An optimal sequence alignment for two sequences is created by aligning the two sequences, *e.g.*, a reference sequence and another sequence, to maximize the number of nucleotide matches in the sequence alignment with appropriate internal nucleotide insertions, deletions, or gaps. As used herein, the term "reference sequence" may refer to a DNA sequence comprising one or more of SEQ ID NOs:1-15.

As used herein, the term "percent sequence identity" or "percent identity" or "% identity" is the identity fraction of two optimally aligned sequences multiplied by 100. The "identity fraction" for a sequence optimally aligned with a reference sequence is the number of nucleotide matches in the optimal alignment, divided by the total number of nucleotides in the reference sequence (*i.e.*, the total number of nucleotides in the full length of the entire reference sequence). Thus, some embodiments of the present disclosure provide a DNA molecule comprising a regulatory sequence that, when optimally aligned to a reference sequence, such as one of SEQ ID NOs:1-15, has at least 80 percent identity, at least 81 percent identity, at least 82 percent identity, at least 83 percent identity, at least 84 percent identity, at least 85 percent identity, at least 86 percent identity, at least 87 percent identity, at least 88 percent identity, at least 89 percent identity, at least 90 percent identity, at least 91 percent identity, at least 92 percent identity, at least 93 percent identity, at least 94 percent identity, at least 95 percent identity, at least 96 percent identity, at least 97 percent identity, at least 98 percent identity, at least 99 percent identity, or 100 percent identity to the reference sequence. According to present embodiments, the regulatory sequence may be operably linked to a transcribable DNA sequence, which may encode a site-specific recombinase.

### Regulatory Elements

Regulatory elements, such as promoters, leaders (also known as 5´ UTRs), enhancers, introns, and transcription termination regions (or 3´ UTRs), play an integral part in the overall expression of genes in living cells. The term "regulatory element," as used herein, refers to a DNA molecule or sequence or segment of DNA having gene-regulatory activity. The term "gene-regulatory activity," as used herein, refers to the ability to affect the expression of an operably linked transcribable DNA molecule, for instance by affecting the transcription and/or translation of the operably linked transcribable DNA molecule. Regulatory elements, such as promoters, leaders, enhancers, introns and 3´ UTRs that function in plants are useful for modifying plant phenotypes through genetic engineering. According to embodiments of the present disclosure, a regulatory element is a promoter having a sequence comprising SEQ ID NO: 2, 7, or 11, or a sequence having at least 80 percent identity, at least 81 percent identity, at least 82 percent identity, at least 83 percent identity, at least 84 percent identity, at least 85 percent identity, at least 86 percent identity, at least 87 percent identity, at least 88 percent identity, at least 89 percent identity, at least 90 percent identity, at least 91 percent identity, at least 92 percent identity, at least 93 percent identity, at least 94 percent identity, at least 95 percent identity, at least 96 percent identity, at least 97 percent identity, at least 98 percent identity, at least 99 percent identity, or 100 percent identity to SEQ ID NO: 2, 7, or 11, or a functional fragment or portion of any of the foregoing sequences. According to embodiments of the present disclosure, a regulatory element is a leader having a sequence comprising SEQ ID NO: 3, 12, or the leader comprised within SEQ ID NO:7, or a sequence having at least 80 percent identity, at least 81 percent identity, at least 82 percent identity, at least 83 percent identity, at least 84 percent identity, at least 85 percent identity, at least 86 percent identity, at least 87 percent identity, at least 88 percent identity, at least 89 percent identity, at least 90 percent identity, at least 91 percent identity, at least 92 percent identity, at least 93 percent identity, at least 94 percent identity, at least 95 percent identity, at least 96 percent identity, at least 97 percent identity, at least 98 percent identity, at least 99 percent identity or 100 percent identity to SEQ ID NO: 3, 12, or the leader comprised within SEQ ID NO:7, or a functional fragment or portion of any of the foregoing sequences that can affect the expression of an operably linked transcribable DNA sequence.

As used herein, a "fragment" of a promoter (or promoter sequence) or regulatory element comprises a fragment or portion of the promoter (or promoter sequence) or regulatory element, respectively, and a "functional fragment" of a promoter (or promoter sequence) or regulatory element comprises a fragment or portion of the promoter (or promoter sequence) or regulatory element, respectively, that affects, modulates or drives the expression of an operably linked transcribable DNA sequence. According to some embodiments, a "functional fragment" of a promoter (or promoter sequence) or regulatory element affects, modulates or drives expression of an operably linked transcribable DNA sequence in a similar manner as the promoter (or promoter sequence) or regulatory element.

As used herein, a "regulatory expression element group" or "EXP" sequence refers to a group of two or more operably linked regulatory elements, such as enhancers, promoters, leaders, and introns. Such two or more operably linked regulatory elements may typically be present together in the same construct and each operably linked to a transcribable DNA sequence. For example, a regulatory expression element group may be comprised, for instance, of a promoter operably linked 5´ to a leader sequence. EXP's useful in practicing the present embodiments may comprise SEQ ID NO: 1, 6, or 10, and sequences having at least 80 percent identity, at least 81 percent identity, at least 82 percent identity, at least 83 percent identity, at least 84 percent identity, at least 85 percent identity, at least 86 percent identity, at least 87 percent identity, at least 88 percent identity, at least 89 percent identity, at least 90 percent identity, at least 91 percent identity, at least 92 percent identity, at least 93 percent identity, at least 94 percent identity, at least 95 percent identity, at least 96 percent identity, at least 97 percent identity, at least 98 percent identity, at least 99 percent identity or 100 percent identity to SEQ ID NO: 1, 6, or 10.

Regulatory elements may be characterized by their associated gene expression pattern in plants, plant tissues and plant cells, *e.g.*, by their positive and/or negative effects on expression, such as constitutive expression or specific patterns of expression, such as temporal, spatial, developmental, tissue, environmental, physiological, pathological or cell cycle expression, and/or chemically responsive or inducible expression, and any combination thereof, as well as by quantitative or qualitative indications or patterns of expression. As used herein, a "gene expression pattern" is any pattern of transcription of an operably linked DNA molecule into a transcribed RNA molecule resulting in relative levels and abundance of the transcribed RNA molecule in various plant tissues and cells during development. Regulatory elements may comprise an enhancer, promoter, leader, 5´ UTR, intron, and/or 3´ UTR.

As used herein, the term "promoter" refers generally to a DNA molecule, segment or sequence that is involved in recognition and binding of RNA polymerase II and other proteins, such as trans-acting transcription factors, to initiate or regulate transcription. A promoter may be initially isolated from an upstream or 5´ untranslated region (5´ UTR) of a genomic copy of a gene. Alternately, promoters may be synthetically produced or engineered DNA molecules. Promoters may also be chimeric. Chimeric promoters are produced through the fusion of two or more heterologous DNA molecules. Promoters useful in practicing the present embodiments may include promoter elements comprising SEQ ID NO: 2, 7, or 11, or a sequence within any of SEQ ID NOs: 1, 6 and 10, or a sequence having at least 80 percent identity, at least 81 percent identity, at least 82 percent identity, at least 83 percent identity, at least 84 percent identity, at least 85 percent identity, at least 86 percent identity, at least 87 percent identity, at least 88 percent identity, at least 89 percent identity, at least 90 percent identity, at least 91 percent identity, at least 92 percent identity, at least 93 percent identity, at least 94 percent identity, at least 95 percent identity, at least 96 percent identity, at least 97 percent identity, at least 98 percent identity, at least 99 percent identity, or 100 percent identity to SEQ ID NOs: 2, 7, or 11, or a sequence having at least 80 percent identity, at least 81 percent identity, at least 82 percent identity, at least 83 percent identity, at least 84 percent identity, at least 85 percent identity, at least 86 percent identity, at least 87 percent identity, at least 88 percent identity, at least 89 percent identity, at least 90 percent identity, at least 91 percent identity, at least 92 percent identity, at least 93 percent identity, at least 94 percent identity, at least 95 percent identity, at least 96 percent identity, at least 97 percent identity, at least 98 percent identity, at least 99 percent identity, or 100 percent identity to a sequence within any of SEQ ID NOs: 1, 6, and 10, or a functional fragment or portion of any of the foregoing sequences. In specific embodiments, DNA molecules and any variants, fragments, portions or derivatives thereof as described herein, are further defined as comprising promoter activity, *i.e.*, are capable of acting as a promoter in a host cell, such as in a transgenic plant. In still further specific embodiments, a fragment of a promoter sequence may be defined as exhibiting promoter activity possessed by the starting promoter molecule from which it is derived, or a fragment may comprise a "minimal promoter" which provides a basal level of transcription and is comprised of a TATA box or equivalent DNA sequence for recognition and binding of the RNA polymerase II complex for initiation of transcription.

In one embodiment, fragments of a promoter sequence disclosed herein are provided. Promoter fragments may comprise promoter activity, as described above, and may be useful alone or in combination with other promoters and/or promoter fragments, such as in constructing chimeric promoters, or in combination with other expression or regulatory elements and expression or regulatory element fragments. In specific embodiments, fragments of a promoter are provided comprising at least about 50, at least about 75, at least about 95, at least about 100, at least about 125, at least about 150, at least about 175, at least about 200, at least about 225, at least about 250, at least about 275, at least about 300, at least about 500, at least about 600, at least about 700, at least about 750, at least about 800, at least about 900, or at least about 1000 contiguous nucleotides, or longer, of a promoter, promoter sequence or DNA molecule having promoter activity as disclosed herein.

Recombinant DNA molecules or constructs comprising a promoter or regulatory element derived from any of the promoter elements provided as SEQ ID NOs: 2, 7, and 11, or from any sequence within any of SEQ ID NOs: 1, 6, and 10, such as internal or truncated sequences or sequences with 5´ deletions, for example, can be produced using methods known in the art to modify or alter expression, such as by removing element(s) or element portion(s) or non-functional spacer sequence(s), that may have either positive or negative effects on expression; duplicating elements that have positive or negative effects on expression; inserting elements that have positive or negative effects on expression; and/or duplicating or removing elements that have tissue-specific, developmental or cell-specific effects on expression. Any recombinant DNA construct or molecule comprising a promoter or regulatory element derived from any of the promoter elements provided as SEQ ID NO: SEQ ID NOs: 2, 7, or 11, or from any sequence within SEQ ID NO: 1, 6, or 10, comprised of 3´ deletions in which the TATA box element or equivalent sequence thereof and downstream sequence is removed can be used, for example, to make enhancer elements. Further deletions can be made to remove any elements that have positive or negative; tissue-specific; cell-specific; or timing-specific (such as, but not limited to, circadian rhythm or developmental timing) effects on expression. Any of the promoter elements provided as SEQ ID NOs: 2, 7, and 11, or comprised within any of SEQ ID NOs: 1, 6, and 10, and fragments or enhancers derived therefrom, can be used to make chimeric transcriptional regulatory element compositions.

In accordance with the invention, a promoter or promoter fragment may be analyzed for the presence of known promoter elements, *i.e.*, DNA sequence characteristics, such as a TATA box and other known transcription factor binding site motifs. Identification of such known promoter elements may be used by one of skill in the art to design variants of the promoter having a similar expression pattern to the original promoter.

As used herein, the term "leader" refers to a DNA molecule isolated from the untranslated 5´ region (5´ UTR) a gene and defined generally as a nucleotide segment between the transcription start site (TSS) and the protein coding sequence start site. Alternately, leaders may be synthetically produced or engineered DNA elements. A leader can be used as a 5´ regulatory element for modulating expression of an operably linked transcribable DNA sequence. Leader sequences may be used with a heterologous promoter or with their native promoter. Leaders useful in practicing the present embodiments may include SEQ ID NO: 3, 12, or the leader comprised within SEQ ID NO:7, or a sequence having at least 80 percent identity, at least 81 percent identity, at least 82 percent identity, at least 83 percent identity, at least 84 percent identity, at least 85 percent identity, at least 86 percent identity, at least 87 percent identity, at least 88 percent identity, at least 89 percent identity, at least 90 percent identity, at least 91 percent identity, at least 92 percent identity, at least 93 percent identity, at least 94 percent identity, at least 95 percent identity, at least 96 percent identity, at least 97 percent identity, at least 98 percent identity, at least 99 percent identity, or 100 percent identity to SEQ ID NO: 3, 12, or the leader comprised within SEQ ID NO:7, or a functional fragment or portion of any of the foregoing sequences; or any of the leader elements comprised within any of SEQ ID NOs: 1, 6, and 10, or within any sequence having at least 80 percent identity, at least 81 percent identity, at least 82 percent identity, at least 83 percent identity, at least 84 percent identity, at least 85 percent identity, at least 86 percent identity, at least 87 percent identity, at least 88 percent identity, at least 89 percent identity, at least 90 percent identity, at least 91 percent identity, at least 92 percent identity, at least 93 percent identity, at least 94 percent identity, at least 95 percent identity, at least 96 percent identity, at least 97 percent identity, at least 98 percent identity, at least 99 percent identity, or 100 percent identity to SEQ ID NOs: 1, 6, or 10, or functional fragments or portions thereof. In specific embodiments, such DNA sequences may be defined as being able to act as a leader in a host cell, including, for example, a transgenic plant cell. In one embodiment, such sequences are defined as comprising leader activity.

The leader sequences (also referred to as 5´ UTRs) presented as SEQ ID NO: 3, 12, or the leader comprised within SEQ ID NO:7; or any of the leader elements comprised within any of SEQ ID NOs:1, 6, and 10 may be comprised of regulatory elements, and/or may adopt secondary structures that can modulate or have an effect on transcription or translation of an operably linked transcribable DNA sequence. The leader sequences presented as SEQ ID NO: 3, 12, or the leader comprised within SEQ ID NO:7 or any fragment thereof, or any of the leader elements comprised within any of SEQ ID NOs: 1, 6, and 10 or any fragment thereof, can be used in accordance with this disclosure to make chimeric regulatory elements that affect transcription or translation of an operably linked transcribable DNA sequence.

As used herein, the term "intron" refers to a DNA molecule or sequence that may be isolated or identified from a gene and may be defined generally as a region spliced out during messenger RNA (mRNA) processing prior to translation. Alternately, an intron may be a synthetically produced or engineered DNA element. An intron may contain enhancer elements that affect the transcription of operably linked genes or transcribable DNA sequences. An intron may be used as a regulatory element for modulating expression of an operably linked transcribable DNA sequence. A construct may comprise an intron, and the intron may or may not be heterologous with respect to the transcribable DNA sequence. Examples of introns in the art include the rice actin intron and the corn HSP70 intron.

In plants, the inclusion of some introns in gene constructs leads to increased mRNA and protein accumulation relative to constructs lacking the intron. This effect has been termed "intron mediated enhancement" (IME) of gene expression. Introns known to stimulate expression in plants have been identified in maize genes (*e.g.*, tubA1, Adh1, Sh1, and Ubi1), in rice genes (*e.g.*, tpi) and in dicotyledonous plant genes like those from petunia (*e.g.*, rbcS), potato (*e.g.*, st-ls1) and from *Arabidopsis thaliana* (*e.g.*, ubq3 and pat1). It has been shown that deletions or mutations within the splice sites of an intron reduce gene expression, indicating that splicing might be needed for IME. However, IME in dicotyledonous plants has been shown by point mutations within the splice sites of the pat1 gene from *A. thaliana*. Multiple uses of the same intron in one plant has been shown to exhibit disadvantages. In those cases, it is necessary to have a collection of basic control elements for the construction of appropriate recombinant DNA elements. Exemplary introns useful in practicing the present invention are presented as SEQ ID NOs: 4, 8, and 13.

As used herein, the terms "3´ transcription termination sequence," "3´ untranslated region," or "3´ UTR" refer to a DNA sequence that is transcribed into the untranslated region within the 3´ portion of an mRNA molecule as generally understood in the art. The 3´ untranslated region of an mRNA molecule may be generated by specific cleavage and 3´ polyadenylation, also known as formation of a polyA tail. A 3´ UTR may be operably linked to and located downstream of a RNA or protein coding portion of a transcribable DNA sequence and may include a polyadenylation signal and other regulatory elements or signals able to affect transcription, mRNA processing, and/or gene expression. PolyA tails are thought to function in mRNA stability and in initiation of translation. Examples of 3´ transcription termination molecules in the art are the nopaline synthase 3´ region, wheat hsp17 3´ region, pea rubisco small subunit 3´ region, cotton E6 3´ region, and the coixin 3´ UTR.

3´ UTRs typically find beneficial use for the recombinant expression of specific DNA molecules. A weak 3´ UTR has the potential to generate read-through, which may affect the expression of the DNA molecule located in the neighboring expression cassettes. Appropriate control of transcription termination can prevent read-through into DNA sequences (*e.g*., other expression cassettes) localized downstream and can further allow efficient recycling of RNA polymerase to improve gene expression. Efficient termination of transcription (release of RNA Polymerase II from the DNA) is prerequisite for re-initiation of transcription and thereby directly affects the overall transcript level. Subsequent to transcription termination, the mature mRNA is released from the site of synthesis and template transported to the cytoplasm. Eukaryotic mRNAs are accumulated as poly(A) forms *in vivo*, making it difficult to detect transcriptional termination sites by conventional methods. However, prediction of functional and efficient 3´ UTRs by bioinformatics methods is difficult in that there are no conserved DNA sequences that would allow easy prediction of an effective 3´ UTR.

Regulation of gene function through 3´ UTRs is a relatively new field as only recent sequencing technology has provided us with the full landscape of 3´ UTRs across species and cell types. Before sequencing technology was available, detailed functional and mechanistic studies were performed only on a few model 3´ UTRs. Although these model 3´ UTRs have contributed substantially to our understanding of 3´ UTR biology, the conclusions drawn about their regulatory functions have been limited and were focused more on mRNA stability. (Mayr, Christine (2017) Regulation by 3´-Untranslated Regions. Annual Review of Genetics, 51: 171-194) A genome-wide in silico analysis revealed that motifs in the 3´ UTR are primarily conserved on one strand, which is consistent with the 3´ UTR acting to regulate gene expression at the post-transcriptional level (Xie, X. et. al., (2005) Systematic discovery of regulatory motifs in human promoters and 3´ UTRs by comparison of several mammals, Nature 434: 338-345). 3´ UTRs determine protein levels through regulation of mRNA stability and translation mediated largely by AU-rich elements and miRNAs. 3´ UTRs also enable local translation through the regulation of mRNA localization. A 3´ UTR's length can be regulated by alternative cleavage and polyadenylation. 3´ UTRs mediate protein-protein interactions (PPIs) which has widespread consequences for protein complex formation, protein localization, and protein function. 3´ UTRs regulate gene expression through the binding of RNA-binding proteins (RBPs). RBPs bind to 3´ UTR *cis*-elements and mediate 3´ UTR functions through the recruitment of effector proteins. RBPs cooperate with other bound RBPs to enable functional specificity *in vivo*. The composition of RBPs bound to a 3´ UTR at a given moment is dynamic and can change depending on the local environment, *e.g.*, through addition of posttranslational modifications, local expression of other RBPs, and interactions with membranes and cytoskeletal filaments. RBP binding is also influenced by secondary and tertiary RNA structure formation that regulates accessibility of 3´ UTRs (Mayr, Christine (2017) Regulation by 3´-Untranslated Regions. Annual Review of Genetics, 51: 171-194).

The poly(A) tail results from the addition of a series of adenosine bases to the 3´ end of an RNA molecule. This provides the mRNA with a binding site for a class of regulatory factors called the poly(A) binding proteins (PABP) that have roles in the regulation of gene expression, including mRNA export, stability and decay, and translation. The 5´cap structure of the mRNA and the poly-A tail function synergistically to control mRNA translation. The association of PABPs with the poly(A) tail facilitates an interaction with eIF4F bound to the 5´cap structure, resulting in circularization of the mRNA that promotes translation initiation and ensures ribosome recycling and efficient translation. This interaction also allows inhibition of translation by inhibitor proteins bound to the 3´ UTR (Barret, L et. al. (2012) Regulation of eukaryotic gene expression by the untranslated regions and other non-coding elements. Cell. Mol. Life Sci. 69:3613-3634).

From a practical standpoint, it is typically beneficial that a 3´ UTR used in an expression cassette possesses the following characteristics. First, the 3´ UTR should be able to efficiently and effectively terminate transcription of the transgene and prevent read-through of the transcript into any neighboring DNA sequence, which can be comprised of another expression cassette as in the case of multiple expression cassettes residing in one construct, or the neighboring chromosomal DNA into which the construct has inserted. Second, the 3´ UTR should not cause a reduction in the transcriptional activity imparted by the promoter, leader, enhancers, and introns that are used to drive expression of the DNA sequence. Finally, in plant biotechnology, the 3´ UTR is often used for priming of amplification reactions of reverse transcribed RNA extracted from the transformed plant and used to: (1) assess the transcriptional activity or expression of the expression cassette once integrated into the plant chromosome; (2) assess the copy number of insertions within the plant DNA; and (3) assess zygosity of the resulting seed after breeding. The 3´ UTR is also used in amplification reactions of DNA extracted from the transformed plant to characterize the intactness of the inserted cassette. 3´ UTRs useful in practicing the present invention are presented as SEQ ID NOs:5, 9, 14, and 15.

As used herein, the term "chimeric" refers to a single DNA molecule produced by fusing a first DNA molecule to a second DNA molecule, where neither the first nor the second DNA molecule would normally be found in that configuration, *i.e.* fused to the other. The chimeric DNA molecule is thus a new DNA molecule not otherwise normally found in nature. As used herein, the term "chimeric promoter" refers to a promoter produced through such manipulation of DNA molecules. A chimeric promoter may combine two or more DNA fragments for example, the fusion of a promoter to an enhancer element. Thus, the design, construction, and use of chimeric promoters according to the methods disclosed herein for modulating the expression of operably linked transcribable DNA molecules are encompassed by the present invention.

Chimeric regulatory elements can be designed to comprise various constituent elements which may be operatively linked by various methods known in the art, such as restriction enzyme digestion and ligation, ligation independent cloning, modular assembly of PCR products during amplification, or direct chemical synthesis of the regulatory element, as well as other methods known in the art. The resulting various chimeric regulatory elements can be comprised of the same, or variants of the same, constituent elements but differ in the DNA sequence or DNA sequences that comprise the linking DNA sequence or sequences that allow the constituent parts to be operatively linked. In the invention, the DNA sequences provided as SEQ ID NOs:1-15 may provide regulatory element reference sequences, wherein the constituent elements that comprise the reference sequence may be joined by methods known in the art and may comprise substitutions, deletions, and/or insertions of one or more nucleotides or mutations that naturally occur in bacterial and plant cell transformation.

As used herein, the term "variant" refers to a second DNA molecule, such as a regulatory element, that is in composition similar, but not identical to, a first DNA molecule, and wherein the second DNA molecule still maintains the general functionality, *i.e.* the same or similar expression pattern, for instance through more or less equivalent transcriptional activity, of the first DNA molecule. A variant may be a shorter or truncated version of the first DNA molecule or an altered version of the sequence of the first DNA molecule, such as one with different restriction enzyme sites and/or internal deletions, substitutions, or insertions. A "variant" can also encompass a regulatory element having a nucleotide sequence comprising a substitution, deletion, or insertion of one or more nucleotides of a reference sequence, wherein the derivative regulatory element has more or less or equivalent transcriptional or translational activity than the corresponding parent regulatory molecule. Regulatory element "variants" will also encompass variants arising from mutations that naturally occur in bacterial and plant cell transformation. In the present invention, a polynucleotide sequence provided as SEQ ID NOs:1-15 may be used to create variants that are similar in composition, but not identical to, the DNA sequence of the original regulatory element, while still maintaining the general functionality, *i.e.*, the same or similar expression pattern, of the original regulatory element. Production of such variants of the invention is well within the ordinary skill of the art in light of the disclosure and is encompassed within the scope of the invention.

As used herein, a "transcribable DNA sequence" is any DNA sequence that when operably linked to a promoter can be transcribed into RNA. The transcribed RNA molecule encoded by the transcribable DNA sequence operably linked to the regulatory element(s) provided herein may be translated to produce a protein molecule or may provide an antisense or other functional or regulatory RNA molecule, such as a double-stranded hairpin RNA (dsRNA), a transfer RNA (tRNA), a ribosomal RNA (rRNA), a microRNA (miRNA), a small interfering RNA (siRNA), and the like.

As used herein, the term "protein expression" is any pattern of translation of a transcribed RNA molecule into a protein molecule. Protein expression may be characterized by its temporal, spatial, developmental, or morphological qualities, as well as by its quantitative or qualitative indications or expression patterns.

The efficacy of the modifications, duplications, or deletions described herein on the desired expression aspects of a particular transgene may be tested empirically in stable and transient plant assays, such as those described in the working examples herein, so as to validate the results, which may vary depending upon the changes made and the goal of the change in the starting DNA molecule.

### Constructs

As used herein, the term "construct" means any DNA molecule or vector, or a segment or portion of a DNA molecule, vector or chromosome, derived from any one or more sources and capable of transfection or genomic integration, comprising at least two DNA sequences linked to each other in a functionally operative manner. For example, a construct may comprise two operably linked sequences, such as a regulatory element or promoter operably linked to a coding sequence or transcribable DNA sequence. A construct may be a recombinant DNA construct. An example of a construct that is a linear, recombinant DNA segment is a T-DNA. As used herein, a "vector" refers to a DNA molecule that may contain or comprise a construct of the present disclosure, such as a plasmid, cosmid, virus, phage, or other linear or circular DNA molecule, and a "DNA transformation vector" mean any DNA molecule or vector comprising a recombinant DNA construct that may be used for the purpose of transformation - *i.e.*, for the introduction of a recombinant DNA molecule or construct into a host cell, such as a plant cell. According to some embodiments, a DNA transformation vector may comprise a T-DNA segment bounded by left and/or right border sequences, which may be used for bacteria-mediated transformation, such as *Rhizobium-*mediated or *Agrobacterium*-mediated transformation. A construct typically includes one or more expression cassettes a gene coding sequence or transcribable DNA sequence operably linked to one or more regulatory sequences, such as a promoter, etc. As used herein, an "expression cassette" refers to a DNA sequence comprising at least a transcribable DNA sequence operably linked to one or more regulatory elements, typically at least a promoter and a 3´ UTR.

As used herein, the term "operably linked" refers to a functional relationship between two or more physically joined DNA sequences of a DNA molecule, construct, vector or chromosome comprising a first and second DNA sequence arranged such that the first DNA sequence affects the function or expression of the second DNA sequence. The two DNA sequences may or may not be part of a single contiguous DNA molecule and may or may not be adjacent. For example, a promoter is operably linked to a transcribable DNA sequence if the promoter modulates transcription of the transcribable DNA sequence of interest in a cell. A leader, for example, is operably linked to a transcribable DNA sequence when it is capable of affecting the transcription or translation of the DNA sequence.

The constructs of the invention may be provided, in one embodiment, as double tumor-inducing (Ti) plasmid border constructs that have the right border (RB or AGRtu.RB) and left border (LB or AGRtu.LB) regions of the Ti or Ri plasmid isolated from *Agrobacterium spp.* (e.g., *A. tumefaciens* or *A. rhizogenes*) comprising a T-DNA that, along with transfer molecules provided by the *Agrobacterium* cells, permit the integration of the T-DNA into the genome of a plant cell (*see*, *e.g.*, U.S. Patent 6,603,061). The constructs may also contain the plasmid backbone DNA segments that provide replication function and antibiotic selection in bacterial cells, *e.g.*, an *Escherichia coli* origin of replication such as ori322, a broad host range origin of replication such as oriV or oriRi (See, e.g., Ye et al., Transgenic Research 20(4):773-86, 2011), and a coding region for a selectable marker such as Spec/Strp that encodes for Tn7 aminoglycoside adenyltransferase (*aadA*) conferring resistance to spectinomycin or streptomycin, or a gentamicin (Gm, Gent) selectable marker gene. For plant transformation, the host bacterial strain is often *A. tumefaciens* ABI, C58, or LBA4404, however other strains known to those skilled in the art of plant transformation can function in the invention.

Methods are known in the art for assembling and introducing constructs into a cell in such a manner that the transcribable DNA molecule is transcribed into a functional mRNA molecule that is translated and expressed as a protein. For the practice of the invention, conventional compositions and methods for preparing and using constructs and host cells are well known to one skilled in the art. Typical vectors useful for expression of nucleic acids in higher plants are well known in the art and include vectors derived from the Ti plasmid of Agrobacterium tumefaciens and the pCaMVCN transfer control vector.

Various regulatory elements may be included in a construct, including any of those provided herein. Any such regulatory elements may be provided in combination with other regulatory elements. Such combinations can be designed or modified to produce desirable regulatory features. In one embodiment, constructs of the invention comprise at least one regulatory element operably linked to a transcribable DNA molecule operably linked to a 3´ UTR.

Constructs of the invention may include any promoter or leader provided herein or known in the art. For example, a promoter of the invention may be operably linked to a heterologous non-translated 5´ leader such as one derived from a heat shock protein gene. Alternatively, a leader of the invention may be operably linked to a heterologous promoter such as the Cauliflower mosaic virus 35S transcript promoter.

Expression cassettes may also include a transit peptide coding sequence that encodes a peptide that is useful for sub-cellular targeting of an operably linked protein, particularly to a chloroplast, leucoplast, or other plastid organelle; mitochondria; peroxisome; vacuole; or an extracellular location. Many chloroplast-localized proteins are expressed from nuclear genes as precursors and are targeted to the chloroplast by a chloroplast transit peptide (CTP). Examples of such isolated chloroplast proteins include, but are not limited to, those associated with the small subunit (SSU) of ribulose-1,5,-bisphosphate carboxylase, ferredoxin, ferredoxin oxidoreductase, the light-harvesting complex protein I and protein II, thioredoxin F, and enolpyruvyl shikimate phosphate synthase (EPSPS). Chloroplast transit peptides are described, for example, in U.S. Patent No. 7,193,133. It has been demonstrated that non-chloroplast proteins may be targeted to the chloroplast by the expression of a heterologous CTP operably linked to the transgene encoding a non-chloroplast protein.

### Transcribable DNA Sequences

As used herein, the term "transcribable DNA sequence" refers to any DNA sequence capable of being transcribed into an RNA molecule, including, but not limited to, those having protein coding sequences and those producing RNA molecules having sequences useful for gene suppression. The type of DNA sequence can include, but is not limited to, a DNA sequence from the same plant, a DNA sequence from another plant, a DNA sequence from a different organism, or a synthetic DNA sequence, such as a DNA sequence containing an antisense message of a gene, or a DNA sequence encoding an artificial, synthetic, or otherwise modified version of a transgene. Exemplary transcribable DNA sequences for incorporation into constructs of the invention include, *e.g.*, DNA sequences or genes from a species other than the species into which the DNA sequence is incorporated or genes that originate from, or are present in, the same species, but are incorporated into recipient cells by genetic engineering methods rather than classical breeding techniques.

A "transgene" refers to a transcribable DNA sequence heterologous to a host cell at least with respect to its location in the host cell genome and/or a transcribable DNA sequence artificially incorporated into a host cell's genome in the current or any prior generation of the cell.

A regulatory element, such as a promoter of the invention, may be operably linked to a transcribable DNA sequence that is heterologous with respect to the regulatory element. As used herein, the term "heterologous" refers to the combination of two or more DNA sequences when such a combination is not normally found in nature. For example, the two DNA sequences may be derived from different species and/or the two DNA sequences may be derived from different genes, *e.g.,* different genes from the same species or the same genes from different species. A regulatory element is thus heterologous with respect to an operably linked transcribable DNA sequence if such a combination is not normally found in nature, *i.e.*, the transcribable DNA sequence does not naturally occur operably linked to the regulatory element. By "heterologous transcribable DNA sequence," it is meant that the transcribable DNA sequence is heterologous with respect to the polynucleotide sequence to which it is operably linked.

The transcribable DNA sequence may generally be any DNA sequence for which expression of a transcript is desired. Such expression of a transcript may result in translation of the resulting mRNA molecule, and thus protein expression. Alternatively, for example, a transcribable DNA sequence may be designed to ultimately cause decreased expression of a specific gene or protein. In one embodiment, this may be accomplished by using a transcribable DNA sequence that is oriented in the antisense direction. One of ordinary skill in the art is familiar with using such antisense technology. Any gene may be negatively regulated in this manner, and, in one embodiment, a transcribable DNA sequence may be designed for suppression of a specific gene through expression of a dsRNA, siRNA or miRNA molecule.

Thus, one embodiment of the invention is a recombinant DNA molecule comprising a regulatory element of the invention, such as those provided as SEQ ID NOs:1-15 or fragment thereof, or a sequence having at least 80 percent identity, at least 81 percent identity, at least 82 percent identity, at least 83 percent identity, at least 84 percent identity, at least 85 percent identity, at least 86 percent identity, at least 87 percent identity, at least 88 percent identity, at least 89 percent identity, at least 90 percent identity, at least 91 percent identity, at least 92 percent identity, at least 93 percent identity, at least 94 percent identity, at least 95 percent identity, at least 96 percent identity, at least 97 percent identity, at least 98 percent identity, at least 99 percent identity, or 100 percent identity to any of SEQ ID NOs:1-15 or fragment thereof, operably linked to a heterologous transcribable DNA sequence so as to modulate transcription of the transcribable DNA sequence at a desired level or in a desired pattern when the construct is integrated in the genome of a transgenic plant cell. In one embodiment, the transcribable DNA sequence comprises a protein-coding region of a gene and in another embodiment the transcribable DNA sequence comprises an antisense region of a gene or any other transcribable DNA sequence that causes suppression of a specific target gene(s).

### Genes of Agronomic Interest

A transcribable DNA sequence may be a gene of agronomic interest. As used herein, the term "gene of agronomic interest" or "transgene of agronomic interest" refers to a transcribable DNA sequence that, when expressed in a particular plant tissue, cell, or cell type, confers a desirable characteristic or trait. The product of a gene or transgene of agronomic interest may act within a plant to cause an effect upon the plant morphology, physiology, growth, development, yield, grain composition, nutritional profile, disease or pest resistance, and/or environmental or chemical tolerance or may act as a pesticidal agent in the diet of a pest that feeds on the plant. In one embodiment of the invention, a regulatory element of the invention is incorporated into a construct such that the regulatory element is operably linked to a transcribable DNA sequence that is a gene or transgene of agronomic interest. In a transgenic plant containing such a construct, the expression of the gene of agronomic interest can confer a beneficial agronomic trait. A beneficial agronomic trait may include, for example, but is not limited to, herbicide tolerance, insect control, modified or increased yield, disease resistance, pathogen resistance, modified plant growth and development, modified starch content, modified oil content, modified fatty acid content, modified protein content, modified fruit ripening, enhanced animal and human nutrition, biopolymer productions, environmental stress tolerance or resistance, pharmaceutical peptides, improved processing qualities, improved flavor, hybrid seed production utility, improved fiber production, and desirable biofuel production.

Non-limiting examples of genes (or transgenes) of agronomic interest known in the art include those for herbicide resistance (U.S. Patent Nos. 6,803,501; 6,448,476; 6,248,876; 6,225,114; 6,107,549; 5,866,775; 5,804,425; 5,633,435; and 5,463,175), increased yield (U.S. Patent Nos. USRE38,446; 6,716,474; 6,663,906; 6,476,295; 6,441,277; 6,423,828; 6,399,330; 6,372,211; 6,235,971; 6,222,098; and 5,716,837), insect control (U.S. Patent Nos. 6,809,078; 6,713,063; 6,686,452; 6,657,046; 6,645,497; 6,642,030; 6,639,054; 6,620,988; 6,593,293; 6,555,655; 6,538,109; 6,537,756; 6,521,442; 6,501,009; 6,468,523; 6,326,351; 6,313,378; 6,284,949; 6,281,016; 6,248,536; 6,242,241; 6,221,649; 6,177,615; 6,156,573; 6,153,814; 6,110,464; 6,093,695; 6,063,756; 6,063,597; 6,023,013; 5,959,091; 5,942,664; 5,942,658, 5,880,275; 5,763,245; and 5,763,241), fungal disease resistance (U.S. Patent Nos. 6,653,280; 6,573,361; 6,506,962; 6,316,407; 6,215,048; 5,516,671; 5,773,696; 6,121,436; 6,316,407; and 6,506,962), virus resistance (U.S. Patent Nos. 6,617,496; 6,608,241; 6,015,940; 6,013,864; 5,850,023; and 5,304,730), nematode resistance (U.S. Patent No. 6,228,992), bacterial disease resistance (U.S. Patent No. 5,516,671), plant growth and development (U.S. Patent Nos. 6,723,897 and 6,518,488), starch production (U.S. Patent Nos. 6,538,181; 6,538,179; 6,538,178; 5,750,876; 6,476,295), modified oils production (U.S. Patent Nos. 6,444,876; 6,426,447; and 6,380,462), high oil production (U.S. Patent Nos. 6,495,739; 5,608,149; 6,483,008; and 6,476,295), modified fatty acid content (U.S. Patent Nos. 6,828,475; 6,822,141; 6,770,465; 6,706,950; 6,660,849; 6,596,538; 6,589,767; 6,537,750; 6,489,461; and 6,459,018), high protein production (U.S. Patent No. 6,380,466), fruit ripening (U.S. Patent No. 5,512,466), enhanced animal and human nutrition (U.S. Patent Nos. 6,723,837; 6,653,530; 6,5412,59; 5,985,605; and 6,171,640), biopolymers (U.S. Patent Nos. USRE37,543; 6,228,623; and 5,958,745, and 6,946,588), environmental stress resistance (U.S. Patent No. 6,072,103), pharmaceutical peptides and secretable peptides (U.S. Patent Nos. 6,812,379; 6,774,283; 6,140,075; and 6,080,560), improved processing traits (U.S. Patent No. 6,476,295), improved digestibility (U.S. Patent No. 6,531,648) low raffinose (U.S. Patent No. 6,166,292), industrial enzyme production (U.S. Patent No. 5,543,576), improved flavor (U.S. Patent No. 6,011,199), nitrogen fixation (U.S. Patent No. 5,229,114), hybrid seed production (U.S. Patent No. 5,689,041), fiber production (U.S. Patent Nos. 6,576,818; 6,271,443; 5,981,834; and 5,869,720) and biofuel production (U.S. Patent No. 5,998,700).

Alternatively, a gene or transgene of agronomic interest can affect the above mentioned plant characteristics or phenotypes by encoding a RNA molecule that causes a targeted modulation of gene expression of an endogenous gene, for example by antisense (*see*, *e.g*. U.S. Patent 5,107,065); inhibitory RNA ("RNAi," including modulation of gene expression by miRNA-, siRNA-, trans-acting siRNA-, and phased sRNA-mediated mechanisms, *e.g*., as described in published applications U.S. 2006/0200878 and U.S. 2008/0066206, and in U.S. patent application 11/974,469); or cosuppression-mediated mechanisms. The RNA could also be a catalytic RNA molecule (*e.g*., a ribozyme or a riboswitch; *see*, *e.g.*, U.S. 2006/0200878) engineered to cleave a desired endogenous mRNA product. Methods are known in the art for constructing and introducing constructs into a cell in such a manner that the transcribable DNA sequence is transcribed into a RNA molecule that is capable of causing gene suppression.

### Selectable Markers

Selectable marker transgenes may also be used with the regulatory elements of the invention. As used herein the term "selectable marker transgene" refers to any transcribable DNA sequence whose expression in a transgenic plant, tissue or cell, or lack thereof, can be screened for or scored in some way. Selectable marker genes, and their associated selection and screening techniques, for use in the practice of the invention are known in the art and include, but are not limited to, transcribable DNA sequences encoding ß-glucuronidase (GUS), green fluorescent protein (GFP), proteins that confer antibiotic resistance, and proteins that confer herbicide tolerance. Examples of selectable marker transgenes are provided as GOI-CP4-EPSPS (SEQ ID NO:21) used for selection of transformed plants cells through glyphosate selection and GOI-GUS (SEQ ID NO:18), a GUS reporter gene used in the Example below in a transgene expression cassette that is intended to remain in the integrated construct after autoexcision to demonstrate retention of the expression cassette and determine zygosity.

### Site-specific Nucleases

As used herein, the term "genome editing" refers to the modification of a genetic sequence at a target site in a DNA molecule or the genome or chromosome of a living organism or cell, such as the genome of a crop plant for agriculture, by deletion, substitution and/or insertion of a DNA sequence at or near the target site, which can be generated using a site-specific nuclease. "Site-specific integration" or "site-directed integration" are terms used to refer to the insertion of a DNA sequence or construct into the genome or chromosome of a living organism or cell at a target site.

As used herein, the term "site-specific nuclease" refers to a DNA-cutting nuclease enzyme that creates a double-strand break or nick at or near a specific target site or location of a DNA molecule, chromosome or genome.

As used herein, a "target site" for genome editing refers to the location of a polynucleotide sequence within a plant genome that is bound and cleaved by a site-specific nuclease introducing a double stranded break (or single-stranded nick) into the nucleic acid backbone of the polynucleotide sequence and/or its complementary DNA strand. After the break or cut is made, the cell's DNA repair mechanism can recognize and repair the break or nick via non-homologous end-joining (NHEJ) or homology-directed repair and possibly introduce a mutation and/or insertion at the target site as understood in the art.

A site-specific nuclease provided herein may be selected from the group consisting of a zinc-finger nuclease (ZFN), a meganuclease, an RNA-guided endonuclease, such as a CRISPR-associated nuclease, a TALE-endonuclease (TALEN), a recombinase, a transposase, or possibly any other endonuclease. See, *e.g.,* Khandagale, K. et al., "Genome editing for targeted improvement in plants," Plant Biotechnol Rep 10: 327-343 (2016); and Gaj, T. et al., "ZFN, TALEN and CRISPR/Cas-based methods for genome engineering," Trends Biotechnol. 31(7): 397-405 (2013), the contents and disclosures of which are incorporated herein by reference. An expression cassette provided herein may encode a site-specific nuclease. Such an expression cassette may comprise a transcribable DNA sequence encoding the site-specific nuclease operably linked to a plant expressible promoter. In another aspect, a recombinant DNA construct provided herein may comprise at least one, at least two, at least three, at least four, at least five, at least six, at least seven, at least eight, at least nine, or at least ten expression cassettes encoding one or more site-specific nuclease(s).

According to embodiments of the present disclosure, a recombinase may be a serine recombinase attached to a DNA recognition motif, a tyrosine recombinase attached to a DNA recognition motif or other recombinase enzyme known in the art. A recombinase or transposase may be a DNA transposase or recombinase attached to a DNA binding domain. A tyrosine recombinase attached to a DNA recognition motif may be selected from the group consisting of a Cre recombinase, a Flp recombinase, and a Tnp 1 recombinase. According to some embodiments, a Cre recombinase or a Gin recombinase provided herein is tethered to a zinc-finger DNA binding domain. In another embodiment, a serine recombinase attached to a DNA recognition motif provided herein is selected from the group consisting of a PhiC31 integrase, an R4 integrase, and a TP-901 integrase. In another embodiment, a DNA transposase attached to a DNA binding domain provided herein is selected from the group consisting of a TALE-piggyBac and TALE-Mutator.

According to embodiments of the present disclosure, an RNA-guided endonuclease or CRISPR-associated nuclease may be selected from the group consisting of Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, Cas12a (also known as Cpf1), CasX, CasY, and homologs or modified versions thereof, Argonaute (non-limiting examples of Argonaute proteins include *Thermus thermophilus* Argonaute (TtAgo), *Pyrococcus furiosus* Argonaute (PfAgo), *Natronobacterium gregoryi* Argonaute (NgAgo) and homologs or modified versions thereof. According to some embodiments, an RNA-guided endonuclease may be a Cas9 or Cas12a enzyme. In another aspect, a site-specific nuclease provided herein is selected from the group consisting of a Cas9 or a Cas12a enzyme.

For RNA-guided endonucleases or CRISPR-associated nuclease, a guide RNA (gRNA) molecule may be required to direct the endonuclease to a target site in a DNA molecule, chromosome or genome of a plant via base-pairing or hybridization to cause a DSB or nick at or near the target site. The gRNA may be transformed or introduced into a plant cell or tissue (perhaps along with a nuclease, or nuclease-encoding DNA construct) as a recombinant DNA construct comprising a transcribable DNA sequence encoding the guide RNA operably linked to a plant-expressible promoter. As understood in the art, a "guide RNA" may comprise, for example, a CRISPR RNA (crRNA), a single-chain guide RNA (sgRNA), or any other RNA molecule that may guide or direct an endonuclease to a specific target site in the genome. A "single-chain guide RNA" (or "sgRNA") is a RNA molecule comprising a crRNA covalently linked to a tracrRNA by a linker sequence, which may be expressed as a single RNA transcript or molecule. The guide RNA comprises a guide or targeting sequence that is identical or complementary to a target site within the DNA molecule, chromosome or plant genome. A protospacer-adjacent motif (PAM) may be present in the genome immediately adjacent and upstream to the 5´ end of the genomic target site sequence complementary to the targeting sequence of the guide RNA - *i.e.,* immediately downstream (3´) to the sense (+) strand of the genomic target site (relative to the targeting sequence of the guide RNA) as known in the art. See, *e.g.*, Wu, X. et al., "Target specificity of the CRISPR-Cas9 system," Quant Biol. 2(2): 59-70 (2014), the content and disclosure of which is incorporated herein by reference. The genomic PAM sequence on the sense (+) strand adjacent to the target site (relative to the targeting sequence of the guide RNA) may comprise 5´-NGG-3´. However, the corresponding sequence of the guide RNA (*i.e.,* immediately downstream (3´) to the targeting sequence of the guide RNA) may generally not be complementary to the genomic PAM sequence. The guide RNA may typically be a non-coding RNA molecule that does not encode a protein. The guide sequence of the guide RNA may be at least 10 nucleotides in length, such as 12-40 nucleotides, 12-30 nucleotides, 12-20 nucleotides, 12-35 nucleotides, 12-30 nucleotides, 15-30 nucleotides, 17-30 nucleotides, or 17-25 nucleotides in length, or about 12, 13, 14, 15, 16, 17, 18, 19, 20, 21, 22, 23, 24, 25 or more nucleotides in length. The guide sequence may be at least 95%, at least 96%, at least 97%, at least 99% or 100% identical or complementary to at least 10, at least 11, at least 12, at least 13, at least 14, at least 15, at least 16, at least 17, at least 18, at least 19, at least 20, at least 21, at least 22, at least 23, at least 24, at least 25, or more consecutive nucleotides of a DNA sequence at the target site. An expression cassette provided herein may encode a guide RNA. Such an expression cassette may comprise a transcribable DNA sequence encoding the guide RNA operably linked to a plant expressible promoter. In another aspect, a recombinant DNA construct provided herein may comprise at least one, at least two, at least three, at least four, at least five, at least six, at least seven, at least eight, at least nine, or at least ten expression cassettes encoding one or more guide RNA(s).

Zinc finger nucleases (ZFNs) are synthetic proteins consisting of an engineered zinc finger DNA-binding domain fused to a cleavage domain (or a cleavage half-domain), which may be derived from a restriction endonuclease (*e.g., Fok1*)*.* The DNA binding domain may be canonical (C2H2) or non-canonical *(e.g.,* C3H or C4). The DNA-binding domain can comprise one or more zinc fingers (*e.g.,* 2, 3, 4, 5, 6, 7, 8, 9 or more zinc fingers) depending on the target site. Multiple zinc fingers in a DNA-binding domain may be separated by linker sequence(s). ZFNs can be designed to cleave almost any stretch of double-stranded DNA by modification of the zinc finger DNA-binding domain. ZFNs form dimers from monomers composed of a non-specific DNA cleavage domain (*e.g.,* derived from the *FokI* nuclease) fused to a DNA-binding domain comprising a zinc finger array engineered to bind a target site DNA sequence. The DNA-binding domain of a ZFN may typically be composed of 3-4 (or more) zinc-fingers. The amino acids at positions -1, +2, +3, and +6 relative to the start of the zinc finger a-helix, which contribute to site-specific binding to the target site, can be changed and customized to fit specific target sequences. The other amino acids may form a consensus backbone to generate ZFNs with different sequence specificities. Methods and rules for designing ZFNs for targeting and binding to specific target sequences are known in the art. See, *e.g.,* US Patent App. Nos. 2005/0064474, 2009/0117617, and 2012/0142062, the contents and disclosures of which are incorporated herein by reference. The *Fok1* nuclease domain may require dimerization to cleave DNA and therefore two ZFNs with their C-terminal regions are needed to bind opposite DNA strands of the cleavage site (separated by 5-7 bp). The ZFN monomer can cut the target site if the two-ZF-binding sites are palindromic. A ZFN, as used herein, is broad and includes a monomeric ZFN that can cleave double stranded DNA without assistance from another ZFN. The term ZFN may also be used to refer to one or both members of a pair of ZFNs that are engineered to work together to cleave DNA at the same site.

Without being limited by any scientific theory, because the DNA-binding specificities of zinc finger domains can be re-engineered using one of various methods, customized ZFN s can theoretically be constructed to target nearly any target sequence (e.g., at or near a GA oxidase gene in a plant genome). Publicly available methods for engineering zinc finger domains include Context-dependent Assembly (CoDA), Oligomerized Pool Engineering (OPEN), and Modular Assembly. In an aspect, a method and/or composition provided herein comprises one or more, two or more, three or more, four or more, or five or more ZFNs. In another aspect, a ZFN provided herein can generate a targeted DSB or nick. In an aspect, vectors comprising polynucleotides encoding one or more, two or more, three or more, four or more, or five or more ZFNs are provided to a cell by transformation methods known in the art (*e.g.,* without being limiting, viral transfection, particle bombardment, PEG-mediated protoplast transfection, or *Agrobacterium-mediated* transformation). The ZFNs may be introduced as ZFN proteins, as polynucleotides encoding ZFN proteins, and/or as combinations of proteins and protein-encoding polynucleotides.

Meganucleases, which are commonly identified in microbes, such as the LAGLIDADG family of homing endonucleases, are unique enzymes with high activity and long recognition sequences (> 14 bp) resulting in site-specific digestion of target DNA. Engineered versions of naturally occurring meganucleases typically have extended DNA recognition sequences (for example, 14 to 40 bp). According to some embodiments, a meganuclease may comprise a scaffold or base enzyme selected from the group consisting of *I-CreI, I-CeuI, I-MsoI, I-SeeI, I-AniI,* and *I-DmoI.* The engineering of meganucleases can be more challenging than ZFNs and TALENs because the DNA recognition and cleavage functions of meganucleases are intertwined in a single domain. Specialized methods of mutagenesis and high-throughput screening have been used to create novel meganuclease variants that recognize unique sequences and possess improved nuclease activity. Thus, a meganuclease may be selected or engineered to bind to a genomic target sequence in a plant. In an aspect, a method and/or composition provided herein comprises one or more, two or more, three or more, four or more, or five or more meganucleases. In another aspect, a meganuclease can generate a targeted cut or break.

Zinc finger nucleases (ZFNs) and TAL effector nucleases (TALENs) are chimeric enzymes that combine a nuclease and a DNA-binding domain. TALENs are a class of sequence-specific nucleases that can be used to make double-stranded breaks at specific target sequences in the genome of a plant or other organism. TALENs are restriction enzymes generated by fusing the transcription activator-like effector (TALE) DNA binding domain to a nuclease domain (e.g., *FokI*)*.* When each member of a TALEN pair binds to the DNA sites flanking a target site, the *FokI* monomers dimerize and cause a double-stranded DNA break at the target site. Besides the wild-type *FokI* cleavage domain, variants of the *FokI* cleavage domain with mutations have been designed to improve cleavage specificity and cleavage activity. The *FokI* domain functions as a dimer, requiring two constructs with unique DNA binding domains for sites in the target genome with proper orientation and spacing. Both the number of amino acid residues between the TALEN DNA binding domain and the *FokI* cleavage domain and the number of bases between the two individual TALEN binding sites are parameters for achieving high levels of activity. TALENs are artificial restriction enzymes generated by fusing the transcription activator-like effector (TALE) DNA binding domain to a nuclease domain. In some aspects, the nuclease is selected from a group consisting of *PvuII, MutH, TevI, FokI, AlwI, MlyI, SbfI, SdaI, StsI, CleDORF, Clo051,* and *Pept071.* When each member of a TALEN pair binds to the DNA sites flanking a target site, the *FokI* monomers dimerize and cause a double-stranded DNA break at the target site. The term TALEN, as used herein, is broad and includes a monomeric TALEN that can cleave double stranded DNA without assistance from another TALEN. The term TALEN also refers to one or both members of a pair of TALENs that work together to cleave DNA at the same site.

Transcription activator-like effectors (TALEs) can be engineered to bind practically any DNA sequence, such as at or near the genomic locus of a GA oxidase gene in a plant. TALE has a central DNA-binding domain composed of 13-28 repeat monomers of 33-34 amino acids. The amino acids of each monomer are highly conserved, except for hypervariable amino acid residues at positions 12 and 13. The DNA-binding domain of TAL effectors may contain 33-35 amino acid sequence repeats which include a repeat-variable di-residue (RVD) at residues 12 and 13, determining their specificity in DNA binding. Each repeat binds a specific nucleotide which has facilitated the engineering of specific DNA-binding domains by selecting a combination of repeat segments containing the appropriate RVD. The number of repeats of the sequence of the RVD determine the length and sequence of the target sequence that will be recognized (Podevin et al. (2013) Trends in Biotechnology 31(6): 375-383). The two variable amino acids are called repeat-variable diresidues (RVDs). The amino acid pairs of RVDs preferentially recognize certain nucleotide bases, and modulation of RVDs can recognize consecutive DNA bases. This simple relationship between amino acid sequence and DNA recognition has allowed for the engineering of specific DNA binding domains by selecting a combination of repeat segments containing the appropriate RVDs.

Besides the wild-type *FokI* cleavage domain, variants of the *FokI* cleavage domain with mutations have been designed to improve cleavage specificity and cleavage activity. The *FokI* domain functions as a dimer, requiring two constructs with unique DNA binding domains for sites in the target genome with proper orientation and spacing. Both the number of amino acid residues between the TALEN DNA binding domain and the *FokI* cleavage domain and the number of bases between the two individual TALEN binding sites are parameters for achieving high levels of activity. *PvuII MutH* and *TevI* cleavage domains are useful alternatives to *FokI* and *FokI* variants for use with TALEs. *PvuII* functions as a highly specific cleavage domain when coupled to a TALE *(see* Yank et al. 2013. PLoS One. 8: e82539). *MutH* is capable of introducing strand-specific nicks in DNA (*see* Gabsalilow et al. 2013. Nucleic Acids Research. 41: e83). *Tevl* introduces double-stranded breaks in DNA at targeted sites (*see* Beurdeley et al., 2013. Nature Comm. 4: 1762). The relationship between amino acid sequence and DNA recognition of the TALE binding domain allows for designable proteins. Software programs such as DNA Works can be used to design TALE constructs. Other methods of designing TALE constructs are known to those of skill in the art. *See* Doyle et al., Nucleic Acids Research (2012) 40: Wl 17-122.; Cermak et al., Nucleic Acids Research (2011). 39:e82; and tale-nt.cac.comell.edu/about. In an aspect, a recombinant DNA construct provided herein comprises one or more, two or more, three or more, four or more, or five or more TALENs. In another aspect, a TALEN provided herein is capable of generating a targeted cut or break at a target site.

Zinc finger nucleases (ZFNs) comprise a zinc finger DNA binding domain and a double-break-inducing domain. Recognition site specificity is conferred by the zinc finger domain, which may comprise two, three, or four zinc fingers, for example having a C2H2 structure, although other zinc finger structures are known and have been engineered. Zinc finger domains an be amenable for designing polypeptides which specifically bind a selected polynucleotide recognition sequence. ZFNs consist of an engineered DNA-binding zinc finger domain linked to a non-specific endonuclease domain, for example nuclease domain from a Type IIs endonuclease, such as FokI. Additional functionalities can be fused to the zinc-finger binding domain, including transcriptional activator domains, transcriptions repressor domains, and methylases. In some examples, dimerization of nuclease domain is required for cleavage activity. Each zinc finger recognizes three consecutive base pairs in the target DNA. For example, a three-finger domain recognizes a sequence of nine contiguous nucleotides, with a dimerization requirement of the nuclease, two sets of zinc finger triplets are used to bind an eighteen-nucleotide recognition sequence (Gaj et al. (2013) Trends Biotechnology, 31(7): 397-405; and Urnov et al. (2010) Nature Reviews Genetics, 11: 636-646).

### Cell Transformation

The invention is also directed to a method of producing transformed cells and plants that comprise one or more regulatory elements operably linked to a transcribable DNA sequence.

The term "transformation" refers to the introduction of a DNA molecule into a recipient host. As used herein, the term "host" refers to bacteria, fungi, or plants, including any cells, tissues, organs, or progeny of the bacteria, fungi, or plants. Plant tissues and cells of particular interest include protoplasts, calli, roots, tubers, seeds, stems, leaves, seedlings, embryos, and pollen.

As used herein, the term "transformed" refers to a cell, tissue, organ, or organism into which a foreign DNA molecule, such as a construct, has been introduced. The introduced DNA molecule may be integrated into the genomic DNA of the recipient cell, tissue, organ, or organism such that the introduced DNA molecule is inherited by subsequent progeny. A "transgenic" or "transformed" cell or organism may also include progeny of the cell or organism and progeny produced from a breeding program employing such a transgenic organism as a parent in a cross and exhibiting an altered phenotype resulting from the presence of a foreign DNA molecule. The introduced DNA molecule may also be transiently introduced into the recipient cell such that the introduced DNA molecule is not inherited by subsequent progeny. The term "transgenic" refers to a bacterium, fungus, or plant containing one or more heterologous DNA molecules.

There are many methods well known to those of skill in the art for introducing DNA molecules into and transforming plant cells. The process generally comprises the steps of selecting a suitable host cell or explant, transforming the cell or explant with a molecule or vector, and obtaining a transformed cell. Methods and materials for transforming plant cells by introducing a plant construct into a plant genome in the practice of this invention can include any of the well-known and demonstrated methods. Suitable methods include, but are not limited to, bacterial infection (*e.g.*, *Agrobacterium*), binary BAC vectors, direct delivery of DNA (*e.g.*, by PEG-mediated transformation, desiccation/inhibition-mediated DNA uptake, electroporation, agitation with silicon carbide fibers, and acceleration of DNA coated particles), gene editing (*e.g.*, CRISPR-Cas systems), among others. According to certain embodiments, methods of transformation include *Agrobacterium* or *Rhizobium* mediated transformation or particle bombardment or microprojectile mediated transformation.

Host cells may be any cell or organism, such as a plant cell, algal cell, algae, fungal cell, fungi, bacterial cell, or insect cell. In specific embodiments, the host cells and transformed cells may include cells from crop plants.

A transgenic plant subsequently may be regenerated from a transgenic plant cell of the invention. Using conventional breeding techniques or self-pollination, seed may be produced from this transgenic plant. Such seed, and the resulting progeny plant grown from such seed, will contain the recombinant DNA molecule of the invention, and therefore will be transgenic.

Transgenic plants of the invention can be self-pollinated to provide seed for homozygous transgenic plants of the invention (homozygous for the recombinant DNA molecule) or crossed with non-transgenic plants or different transgenic plants to provide seed for heterozygous transgenic plants of the invention (heterozygous for the recombinant DNA molecule). Both such homozygous and heterozygous transgenic plants are referred to herein as "progeny plants." Progeny plants are transgenic plants descended from the original transgenic plant and containing the recombinant DNA molecule of the invention. Seeds produced using a transgenic plant of the invention can be harvested and used to grow generations of transgenic plants, *i.e.*, progeny plants of the invention, comprising the construct of this invention and expressing a gene of agronomic interest. Descriptions of breeding methods that are commonly used for different crops can be found in one of several reference books, *see, e.g.*, Allard, Principles of Plant Breeding, John Wiley & Sons, NY, U. of CA, Davis, CA, 50-98 (1960); Simmonds, Principles of Crop Improvement, Longman, Inc., NY, 369-399 (1979); Sneep and Hendriksen, Plant breeding Perspectives, Wageningen (ed), Center for Agricultural Publishing and Documentation (1979); Fehr, Soybeans: Improvement, Production and Uses, 2nd Edition, Monograph, 16:249 (1987); Fehr, Principles of Variety Development, Theory and Technique, (Vol. 1) and Crop Species Soybean (Vol. 2), Iowa State Univ., Macmillan Pub. Co., NY, 360-376 (1987).

The transformed plants may be analyzed for the presence of the gene or genes of interest and the expression level and/or profile conferred by the regulatory elements of the invention. Those of skill in the art are aware of the numerous methods available for the analysis of transformed plants. For example, methods for plant analysis include, but are not limited to, Southern blots or northern blots, PCR-based approaches, biochemical analyses, phenotypic screening methods, field evaluations, and immunodiagnostic assays. The expression of a transcribable DNA sequence can be measured using TaqMan^{®} (Applied Biosystems, Foster City, CA) reagents and methods as described by the manufacturer and PCR cycle times determined using the TaqMan^{®} Testing Matrix. Alternatively, the Invader^{®} (Third Wave Technologies, Madison, WI) reagents and methods as described by the manufacturer can be used to evaluate transgene expression.

The invention also provides for parts of a plant of the invention. Plant parts include, but are not limited to, leaves, stems, roots, tubers, seeds, endosperm, ovule, and pollen. Plant parts of the invention may be viable, nonviable, regenerable, and/or non-regenerable. The invention also includes and provides transformed plant cells comprising a DNA molecule of the invention. The transformed or transgenic plant cells of the invention include regenerable and/or non-regenerable plant cells.

The invention also provides a commodity product that is produced from a transgenic plant or part thereof containing the recombinant DNA molecule of the invention. Commodity products of the invention contain a detectable amount of DNA comprising a DNA sequence selected from the group consisting of SEQ ID NOs:1-15. As used herein, a "commodity product" refers to any composition or product which is comprised of material derived from a transgenic plant, seed, plant cell, or plant part containing the recombinant DNA molecule of the invention. Commodity products include but are not limited to processed seeds, grains, plant parts, and meal. A commodity product of the invention will contain a detectable amount of DNA corresponding to the recombinant DNA molecule of the invention. Detection of one or more of this DNA in a sample may be used for determining the content or the source of the commodity product. Any standard method of detection for DNA molecules may be used, including methods of detection disclosed herein.

The invention may be more readily understood through reference to the following examples, which are provided by way of illustration, and are not intended to be limiting of the invention, unless specified. It should be appreciated by those of skill in the art that the techniques disclosed in the following examples represent techniques discovered by the inventors to function well in the practice of the invention. However, those of skill in the art should, in light of the present disclosure, appreciate that many changes can be made in the specific embodiments that are disclosed and still obtain a like or similar result without departing from the spirit and scope of the invention, therefore all matter set forth is to be interpreted as illustrative and not in a limiting sense.

### EXAMPLES

### Example 1

### Identification of Regulatory Elements Able to Drive Autoexcision in Crop Plants

This example presents the regulatory elements that have been identified over many years of experimentation that are able to drive efficient autoexcision in transgenic corn, soybean, and cotton.

The regulatory elements with the potential to drive efficient autoexcision in transgenic crop plants were first identified through a combination of literature searches and searches of public and proprietary databases. Over one-hundred corn binary transformation vector constructs comprising different regulatory elements and combinations have been assayed for efficient autoexcision using the Cre/Lox recombinase system. From these studies, a small number of regulatory elements were identified that provided efficient autoexcision and are presented in Table 1 below.

**Table 1. Regulatory elements that provide efficient autoexcision in crop plants.**

| **Description** | **SEQ ID NO:** | **Description and/or regulatory elements of EXP linked in 5´ → 3´ direction (SEQ ID NOs:)** |
|---|---|---|
| EXP-Zm.BabyBoom2 | 1 | EXP: P-Zm.BabyBoom2:2 (SEQ ID NO:2); L-Zm.BabyBoom2:1 (SEQ ID NO:3); I-Zm.BabyBoom2:1 (SEQ ID NO:4) |
| P-Zm.BabyBoom2:2 | 2 | Promoter |
| L-Zm.BabyBoom2:1 | 3 | Leader |
| I-Zm.BabyBoom2:1 | 4 | Intron |
| T-Zm.BabyBoom2:1 | 5 | 3´ UTR |
| EXP-Zm.GRMZM2G512113_GSI85 | 6 | EXP: P-Zm.GRMZM2G512113:1 (SEQ ID NO:7); I-Zm.GSI85.nno:1 (SEQ ID NO:8) |
| P-Zm.GRMZM2G512113:1 | 7 | Promoter + Leader |
| I-Zm.GSI85.nno:1 | 8 | Intron |
| T-Zm.GRMZM2G512113:1 | 9 | 3´ UTR |
| EXP-SETvi.SPO11-1_Eef7 | 10 | EXP: P-SETvi.SPO11-1:1 (SEQ ID NO:11); L-SETvi.SPO11-1:1 (SEQ ID NO:12); I-SETit.Eef7:2 (SEQ ID NO:13) |
| P-SETvi.SPO11-1:1 | 11 | Promoter |
| L-SETvi.SPO11-1:1 | 12 | Leader |
| I-SETit.Eef7:2 | 13 | Intron |
| T-SETvi.SPO11-1:1 | 14 | 3´ UTR |
| T-SACra.Hsp16.9:29 | 15 | 3´ UTR |

### Example 2

### The Zea mays BabyBoom2 and Zm.GRMZM2G512113 EXPs is able to Drive Autoexcision in Stably Transformed Corn Plants

Corn plants were transformed with recombinant DNA constructs, specifically plant transformation constructs, comprising different regulatory elements driving expression of a Cre-recombinase to assess the ability and efficiency of the Cre-recombinase expressed under the control of the different regulatory elements in driving autoexcision of the Cre-recombinase expression cassette along with multiple expression cassettes.

Corn plants were transformed with binary plant transformation constructs comprising 5 transgene expression cassettes: a Cre-recombinase expression cassette, a selectable marker gene expression cassette, an expression cassette used for the expression of Cas12a, and an expression cassette used for expression of a guide RNA (gRNA) all flanked by two LoxP sites (SEQ ID NO:20); and a fifth expression cassette located outside of the LoxP sites that expresses a ß-glucuronidase (GUS) transgene. The Cre-recombinase expression cassette was used to assay different EXP's to test for their ability to drive efficient autoexcision of the Cre-recombinase expression cassette, marker gene expression cassette, Cas12a expression cassette, and the gRNA expression cassette located between the LoxP sites. The Cre-recombinase expression cassette was comprised of an EXP to be tested, operably linked 5´ to a synthetic coding sequence (*e.g.*, a codon redesigned for expression in a plant cell) encoding a Cre-recombinase (GOI-Cre_1, SEQ ID NO:16) containing a processable intron derived from the potato light-inducible tissue-specific ST-LS1 gene (GenBank Accession: X04753), operably linked 5´ to a 3´ UTR. Each plant transformation construct comprised a marker gene expression cassette used for expression of a CP4-EPSPS coding sequence (SEQ ID NO:21), driven by a constitutive promoter, and was used for selection of transformed plant cells using glyphosate selection. Two additional expression cassettes were cloned adjacent to the Cre and CP4 expression cassettes, one used for the expression of Cas12a driven by a constitutive promoter and the other used for the expression of a gRNA driven by a polymerase III promoter.

The 4 expression cassettes, the marker gene expression cassette, the Cre-recombinase expression cassette, the Cas12a expression cassette, and the gRNA expression cassette were flanked by two LoxP Cre-recombinase recognition sequences (SEQ ID NO:20) in a head to tail orientation. Expression of the Cre-recombinase within the transformed plant cell would be expected to result in excision of all four expression cassettes if autoexcision is effective. The GUS expression cassette was cloned outside of the LoxP Cre-recombinase recognition sequences and used a constitutive promoter to drive a coding sequence encoding ß-glucuronidase (GOI-GUS, SEQ ID NO:18) which comprised a processable intron derived from the potato light-inducible tissue-specific ST-LS1 gene (GenBank Accession: X04753).

Two EXPs, EXP-Zm.BabyBoom2 and EXP-Zm.GRMZM2G512113_GSI85 derived from corn genes were operably linked to a transcribable DNA sequence encoding a Cre recombinase (GOI-Cre_1, SEQ ID NO:16) and assayed for their ability to drive autoexcision in stably transformed corn plants. EXP-Zm.BabyBoom2 (SEQ ID NO:1) comprised a promoter P-Zm.BabyBoom2:2 (SEQ ID NO:2), operably linked 5´ to a leader L-Zm.BabyBoom2:1 (SEQ ID NO:3), operably linked 5´ to an intron (I-Zm.BabyBoom2:1, SEQ ID NO:4). EXP-Zm.GRMZM2G512113_GSI85 (SEQ ID NO:6) was comprised of a promoter and leader (P-Zm.GRMZM2G512113:1, SEQ ID NO:7), operably linked 5´ to a synthetic intron (I-Zm.GSI85.nno:1, SEQ ID NO:8). Each EXP/Cre_1 expression cassette comprised a corresponding 3´ UTR (T-Zm.BabyBoom2:1, SEQ ID NO:5 and T-Zm.GRMZM2G512113:1, SEQ ID NO:9) derived from the same gene from which the promoter was derived operably linked 3´ to the Cre_1 (GOI-Cre_1, SEQ ID NO:16) coding sequence within the expression cassette.

Corn plant cells were transformed, using the binary plant transformation constructs described above by *Agrobacterium*-mediated transformation. Methods for *Agrobacterium-*mediated transformation are well known in the art. The resulting transformed plant cells were regenerated into corn plants under glyphosate selection.

Single and two copy R₀ plants were selected and allowed to self-pollinate. The resulting R₁ plants were then analyzed for the presence of the Cre, CP4 and GUS expression cassettes using a TAQMAN^{®} assay. Zygosity of the R₁ plants for the integrated construct was also determined using a TAQMAN^{®} assay for the GUS transgene. Absence of the CP4 cassette was inferred to indicate absence of all 4 cassettes that were cloned between the two LoxP recognition sequences. Table 2 below shows the total number and percent homozygous and hemizygous marker free R₁ seeds from the two different EXPs.

**Table 2. Total number and percent homozygous and hemizygous marker free R₁ plants from the two different EXPs.**

| **EXP SEQ ID NO:** | **3' UTR SEQ ID NO:** | **Total Number Plants** | **Total Number Homozygous Marker Free** | **Total Number Hemizygous Marker Free** | **Percent Homozygous Marker Free** | **Percent Hemizygous Marker Free** |
|---|---|---|---|---|---|---|
| 1 | 5 | 1323 | 13 | 158 | 0.98 | 11.94 |
| 6 | 9 | 859 | 6 | 80 | 0.70 | 9.31 |

As can be seen in Table 2 above, EXP-Zm.BabyBoom2 (SEQ ID NO:1) and EXP-Zm.GRMZM2G512113_GSI85 (SEQ ID NO:6) were able to drive autoexcision, resulting in many hemizygous marker free plants plants and homozygous marker free plants.

### Example 3

### EXP-SETvi.SPO11-1_Eef7 is able to Drive Autoexcision in Stably Transformed Corn Plants

Corn plants were transformed with recombinant DNA constructs, specifically plant transformation constructs, comprising EXP-SETvi.SPO11-1_Eef7 (SEQ ID NO:10) driving expression of a Cre-recombinase to assess the ability and efficiency of the Cre-recombinase expressed under the control of EXP-SETvi.SPO11-1_Eef7 (SEQ ID NO:10) and 2 different 3´ UTRs to drive autoexcision of the Cre-recombinase expression cassette along with the selectable marker expression cassette.

Corn plants were transformed with binary plant transformation constructs comprising 3 transgene expression cassettes: a Cre-recombinase expression cassette, a selectable marker gene expression cassette, flanked by two LoxP sites (SEQ ID NO:20); and a third expression cassette located outside of the LoxP sites that expressed the TIC10746_3 insect toxin transgene (SEQ ID NO:19). The Cre-recombinase expression cassette was used to assay the EXP, EXP-SETvi.SPO11-1_Eef7 (SEQ ID NO:10) for its ability to drive efficient autoexcision of the Cre-recombinase expression cassette and marker gene expression cassette located between the LoxP recognition sites. Two constructs were made with a Cre expression cassette comprising the EXP and one of two different 3´ UTRs T-SETvi.SPO11-1:1 (SEQ ID NO:14) or T-SACra.Hsp16.9:29 (SEQ ID NO:15). The Cre-recombinase expression cassette was comprised of the EXP, EXP-SETvi.SPO11-1_Eef7 (SEQ ID NO:10), operably linked 5´ to a synthetic coding sequence (*e.g.*, a codon redesigned for expression in a plant cell) encoding a Cre-recombinase (GOI-Cre_2, SEQ ID NO:17) containing a processable intron derived from the *Setaria italica* LS8 gene (United States patent application US20190292542, SEQ ID NO:244), operably linked 5´ to one of two 3´ UTRs, T-SETvi.SPO11-1:1 (SEQ ID NO:14) or T-SACra.Hsp16.9:29 (SEQ ID NO:15). Each plant transformation construct comprised a marker gene expression cassette used for expression of a CP4-EPSPS coding sequence (SEQ ID NO:21), driven by a constitutive promoter, and was used for selection of transformed plant cells using glyphosate selection.

The 2 expression cassettes, the marker gene expression cassette and the Cre-recombinase expression cassette were flanked by two LoxP Cre-recombinase recognition sequences (SEQ ID NO:20) in a head to tail orientation. Expression of the Cre-recombinase within the transformed plant cell would be expected to result in excision of the 2 expression cassettes if autoexcision is effective. The TIC10746_3 expression cassette was cloned outside of the LoxP Cre-recombinase recognition sequences and used a constitutive promoter to drive a synthetic coding sequence encoding TIC10746_3.

Corn plant cells were transformed, using the binary plant transformation constructs described above by *Agrobacterium*-mediated transformation. Methods for *Agrobacterium-*mediated transformation are well known in the art. The resulting transformed plant cells were regenerated into corn plants under glyphosate selection.

Single and two copy R₀ plants were selected and allowed to self-pollinate. The resulting R₁ plants were then analyzed for the presence of the Cre, CP4 and TIC10746_3 expression cassettes using a TAQMAN^{®} assay. Zygosity of the R₁ plants for the integrated construct was also determined using a TAQMAN^{®} assay of the TIC10746_3 transgene. Absence of the CP4 cassette was inferred to indicate absence of both cassettes that were cloned between the two LoxP recognition sequences. Table 3 below shows the total number and percent homozygous and hemizygous marker free R₁ plants from the two different constructs.

**Table 3. Total number and percent homozygous and hemizygous marker free R₁ plants from the two different constructs.**

| **EXP SEQ ID NO:** | **3' UTR SEQ ID NO:** | **Total Number Plants** | **Total Number Homozygous Marker Free** | **Total Number Hemizygous Marker Free** | **Percent Homozygous Marker Free** | **Percent Hemizygous Marker Free** |
|---|---|---|---|---|---|---|
| 10 | 14 | 207 | 0 | 56 | 0 | 27.1 |
| 10 | 15 | 399 | 0 | 65 | 0 | 16.3 |

As can be seen in Table 3 above, EXP-SETvi.SPO11-1_Eef7 (SEQ ID NO:10) was able to drive efficient autoexcision using either of the two 3´ UTRs, T-SETvi.SPO11-1:1 (SEQ ID NO:14) or T-SACra.Hsp16.9:29 (SEQ ID NO:15), resulting in many hemizygous marker free R₁ plants from both EXPs.

### Example 4

### EXP-SETvi.SPO11-1_Eef7 is able to Drive Autoexcision in Stably Transformed Corn Plants

Corn plants were transformed with recombinant DNA constructs, specifically plant transformation constructs, comprising EXP-SETvi.SPO11-1_Eef7 (SEQ ID NO:10) driving expression of a Cre-recombinase to assess the ability and efficiency of the Cre-recombinase expressed under the control of EXP-SETvi.SPO11-1_Eef7 (SEQ ID NO:10) and 2 different 3´ UTRs to drive autoexcision of the Cre-recombinase expression cassette along with three other expression cassettes flanked by LoxP sites.

Corn plants were transformed with two binary plant transformation constructs similar to those described in Example 2, comprising 5 transgene expression cassettes: a Cre-recombinase expression cassette, a selectable marker gene expression cassette, an expression cassette used for the expression of Cas12a, and an expression cassette used for expression of a guide RNA (gRNA) all flanked by two LoxP sites (SEQ ID NO:20); and a fifth expression cassette located outside of the LoxP sites that expresses a ß-glucuronidase (GUS) transgene. The Cre-recombinase expression cassette was comprised of the EXP, EXP-SETvi.SPO11-1_Eef7 (SEQ ID NO:10), operably linked 5´ to a synthetic coding sequence (*e.g.*, a codon redesigned for expression in a plant cell) encoding a Cre-recombinase (GOI-Cre_1, SEQ ID NO:16) containing a processable intron derived from the *Setaria italica* LS8 gene (United States patent application US20190292542, SEQ ID NO:244), operably linked 5´ to one of two 3´ UTRs, T-SETvi.SPO11-1:1 (SEQ ID NO:14) or T-SACra.Hsp16.9:29 (SEQ ID NO:15).

Corn plant cells were transformed, using the binary plant transformation constructs described above by *Agrobacterium*-mediated transformation. Methods for *Agrobacterium-*mediated transformation are well known in the art. The resulting transformed plant cells were regenerated into corn plants under glyphosate selection.

Single and two copy R₀ plants were selected and allowed to self-pollinate. The resulting R₁ plants were then analyzed for the presence of the Cre, CP4 and GUS expression cassettes using a TAQMAN^{®} assay. Zygosity of the R₁ plants for the integrated construct was also determined using a TAQMAN^{®} assay for the GUS transgene. Absence of the CP4 cassette was inferred to indicate absence of all 4 cassettes that were cloned between the two LoxP recognition sequences. Table 4 below shows the total number and percent homozygous and hemizygous marker free R₁ seeds from the two different EXPs.

**Table 4. Total number and percent homozygous and hemizygous marker free R₁ plants from the two different constructs.**

| **EXP SEQ ID NO:** | **3' UTR SEQ ID NO:** | **Total Number Plants** | **Total Number Homozygous Marker Free** | **Total Number Hemizygous Marker Free** | **Percent Homozygous Marker Free** | **Percent Hemizygous Marker Free** |
|---|---|---|---|---|---|---|
| 10 | 14 | 1214 | 7 | 133 | 0.58 | 10.96 |
| 10 | 15 | 1236 | 17 | 177 | 1.38 | 14.32 |

As can be seen in Table 4 above, EXP-SETvi.SPO11-1_Eef7 (SEQ ID NO:10) was able to drive efficient autoexcision using either of the two 3´ UTRs, T-SETvi.SPO11-1:1 (SEQ ID NO:14) or T-SACra.Hsp16.9:29 (SEQ ID NO:15), resulting in many hemizygous marker free R₁ plants from both EXPs.

### Example 5

### Assay of EXP-SETvi.SPO11-1_Eef7 Activity in Stably Transformed Corn Plants

Corn plants were transformed with recombinant DNA constructs, specifically plant transformation constructs, comprising EXP-SETvi.SPO11-1_Eef7 (SEQ ID NO:10) and 2 different 3´ UTRs driving expression of a ß-glucuronidase (GUS) transgene. The resulting plants were analyzed for GUS protein expression, to assess the effect of the regulatory element group (EXP) on expression.

Corn plants were transformed with two plant GUS expression constructs. The EXP, EXP-SETvi.SPO11-1_Eef7 (SEQ ID NO:10) was cloned into a base plant expression construct using standard methods known in the art. The resulting plant expression construct contained a left border region from *Agrobacterium tumefaciens*, a first transgene selection cassette used for selection of transformed plant cells that confers resistance to the herbicide glyphosate, a second transgene cassette to assess the activity of EXP-SETvi.SPO11-1_Eef7 comprised of a transgene cassette comprising EXP-SETvi.SPO11-1_Eef7 (SEQ ID NO:10) operably linked 5´ to a coding sequence for GUS (SEQ ID NO:18) comprised of a processable intron, operably linked 5´ to one of two 3´ UTRs, T-SETvi.SPO11-1:1 (SEQ ID NO:14) or T-SACra.Hsp16.9:29 (SEQ ID NO:15), and a right border region from *Agrobacterium tumefaciens*.

Corn plant cells were transformed using the binary transformation vector construct described above by *Agrobacterium*-mediated transformation, as is well known in the art. The resulting transformed plant cells were induced to form whole corn plants.

Qualitative and quantitative GUS analysis was used to evaluate expression element activity in selected plant organs and tissues in transformed plants. For qualitative analysis of GUS expression by histochemical staining, whole-mount or sectioned tissues were incubated with GUS staining solution containing 1 mg/mL of X-Gluc (5-bromo-4-chloro-3-indolyl-b-glucuronide) for 5 h at 37° C and de-stained with 35 % EtOH and 50 % acetic acid. Expression of GUS was qualitatively determined by visual inspection of selected plant organs or tissues for blue coloration under a dissecting or compound microscope.

For quantitative analysis of GUS expression by enzymatic assays, total protein was extracted from selected tissues of transformed corn plants. One to two micrograms of total protein was incubated with the fluorogenic substrate, 4-methyleumbelliferyl-β-D-glucuronide (MUG) at 1 mM concentration in a total reaction volume of 50 microliters. After 1 h incubation at 37° C, the reaction was stopped by adding 350 microliters of 200 mM sodium bicarbonate solution. The reaction product, 4-methlyumbelliferone (4-MU), is maximally fluorescent at high pH, where the hydroxyl group is ionized. Addition of the basic sodium carbonate solution simultaneously stops the assay and adjusts the pH for quantifying the fluorescent product 4-MU. The amount of 4-MU formed was estimated by measuring its fluorescence using a FLUOstar Omega Microplate Reader (BMG LABTECH) (excitation at 355 nm, emission at 460 nm). GUS activity values are provided in nmoles of 4-MU /hour/mg total protein.

The following tissues were sampled for GUS expression in the R₀ generation: V4 stage Leaf and Root; V7 stage Leaf and Root; VT stage Leaf, pollen, Spikelet, and Stem/internode; R1 stage Cob/Silk; and R3 stage Seed 21 days after pollination (DAP). Table 5 shows the mean quantitative GUS expression for the sampled tissues wherein "bdl" indicates below detection level.

**Table 5. Mean GUS expression of stably transformed corn plants.**

| | | **EXP-SETit.Spo11/T-SETit.Spo11** | | **EXP-SETit.Spo11/T-SACra.Hsp16.9** | |
|---|---|---|---|---|---|
| **Stage** | **Organ** | **Range** | **Mean** | **Range** | **Mean** |
| V4 | Leaf | 21 - 77.62 | 38.01 | 20.61 - 156.72 | 56.04 |
| | Root | 24.64 - 60.45 | 39.56 | 24.07 - 95.57 | 47.71 |
| V7 | Leaf | 20.18 - 31.9 | 25.85 | 22.5 - 56.68 | 35.84 |
| | Root | 25.69 - 123.56 | 43.57 | 26.88 - 101.49 | 46.56 |
| VT | Leaf | 20.28 - 51.39 | 29.77 | - | - |
| | Pollen | 20.69 - 256.61 | 120.11 | 30.82 - 517.76 | 234.6 |
| | Spikelet | 22.48 - 61.23 | 38.96 | 29.98 - 184.44 | 88.03 |
| | Stem/internode | bdl | bdl | 20.97 - 24.31 | 22.47 |
| R1 | Cob/silk | 20.49 - 71.17 | 27.98 | 21.12 - 44.57 | 27.88 |
| R3 | Seed 21DAP | 26.5 - 113.07 | 50.97 | 20.02 - 361.73 | 67.63 |

As can be seen in Table 5 above, EXP-SETvi.SPO11-1_Eef7 (SEQ ID NO:10) was able to drive GUS expression in most tissues sampled when operably linked to either of the 3´ UTRs, T-SETvi.SPO11-1:1 (SEQ ID NO:14) or T-SACra.Hsp16.9:29 (SEQ ID NO:15). Expression was highest in VT Pollen and Spikelet. Staining of VT Spikelets from multiple events showed expression to be isolated to the Pollen within the immature Spikelet. The expression pattern conferred by EXP-SETvi.SPO11-1_Eef7 (SEQ ID NO:10) was similar using either of the two 3´ UTRs, T-SETvi.SPO11-1:1 (SEQ ID NO:14) or T-SACra.Hsp16.9:29 (SEQ ID NO:15), but expression was almost 2 fold higher in VT Pollen and almost 2.3 fold higher in VT Spikelet using T-SACra.Hsp16.9:29 when compared to T-SETvi.SPO11-1:1.

### Example 6

### Assay of Intron Mediated Enhancement Activity of I-Zm.GSI85.nno:1 in Stably Transformed Corn Plants

Corn plants were transformed with recombinant DNA constructs, specifically plant transformation constructs, comprising a constitutive promoter operably linked to the intron, I-Zm.GSI85.nno:1 (SEQ ID NO:8) driving expression of a ß-glucuronidase (GUS) transgene and compared to plants expressing GUS driven by the same promoter but lacking the intron to assess the expression enhancement effect of the intron.

Corn plants were transformed with two plant GUS expression constructs, a control construct and experimental construct, which were built using methods known in the art. Both constructs contained a left border region from *Agrobacterium tumefaciens*, a first transgene selection cassette used for selection of transformed plant cells that confers resistance to the herbicide glyphosate, a second transgene cassette used to assess the enhancement activity imparted by the intron, I-Zm.GSI85.nno:1 (SEQ ID NO:8), and a right border region from *Agrobacterium tumefaciens*. The second transgene cassette of the control construct comprised a low expressing constitutive promoter and leader operably linked 5´ to a coding sequence for GUS comprised of a processable intron (SEQ ID NO:18), operably linked 5´ to a 3´ UTR. The second transgene cassette of the experimental construct comprised the same promoter, leader, GUS coding sequence, and 3´ UTR as the control construct and included the intron, I-Zm.GSI85.nno:1, operably linked 3´ to the leader and 5´ to the GUS coding sequence.

Corn plant cells were transformed using the binary transformation vector construct described above by *Agrobacterium*-mediated transformation, as is well known in the art. The resulting transformed plant cells were induced to form whole corn plants.

Qualitative and Quantitative GUS expression was assayed as previously described in Example 5. The following tissues were sampled for GUS expression in the R₀ generation: V4 stage Leaf and Root; V7 stage Leaf and Root; VT stage Flower/anthers, Leaf, and Root; R1 stage Cob/Silk; and R3 stage Seed Embryo and Endosperm 21 days after pollination (DAP). Table 6 shows the mean quantitative GUS expression for the sampled tissues wherein "bdl" indicates below detection level.

**Table 6. Mean GUS expression of stably transformed corn plants.**

| **Stage** | **Organ** | **Without GSI85** | **With GSI85** |
|---|---|---|---|
| V4 | Leaf | bdl | 217.61 |
| | Root | 10.47 | 19.93 |
| V7 | Leaf | 13.29 | bdl |
| | Root | bdl | bdl |
| VT | Flower/anthers | 11.16 | 40.06 |
| | Leaf | bdl | 26.14 |
| | Root | 12.45 | bdl |
| R1 | Cob/silk | 13.15 | bdl |
| R3 | Seed Embryo 21 DAP | bdl | 77.6 |
| | Seed Endosperm 21 DAP | bdl | 19.61 |

As can be seen in Table 6 above, the addition of the intron, I-Zm.GSI85.nno:1 increased expression in several tissues. The greatest enhancement of expression was observed in V4 Leaf and seed Embryo 21 Days After Pollination. Expression was also enhanced in VT Flowers/anthers and Leaf. The intron, I-Zm.GSI85.nno:1 (SEQ ID NO:8) provides intron mediated enhancement when operably linked to a promoter in an expression cassette.

### EMBODIMENTS

For further illustration, additional non-limiting embodiments of the present invention are set forth below.

Embodiment 1 is a recombinant DNA construct comprising a DNA regulatory sequence comprising:
a. a sequence with at least 80% sequence identity to any of SEQ ID NOs:1-15;
b. a sequence comprising any of SEQ ID NOs:1-15; and
c. a fragment of (i) any of SEQ ID NOs:1-15 or (ii) any sequence with at least 80% sequence identity to any of SEQ ID NOs:1-15, wherein the fragment has gene regulatory activity;
wherein said DNA regulatory sequence is operably linked to a heterologous transcribable DNA sequence encoding a site-specific recombinase.

Embodiment 2 is the recombinant DNA construct of embodiment 1, wherein said DNA regulatory sequence has at least 90 percent sequence identity to the DNA sequence of any of SEQ ID NOs:1-15.

Embodiment 3 is the recombinant DNA construct of embodiment 1 or 2, wherein said DNA regulatory sequence has at least 95 percent sequence identity to the DNA sequence of any of SEQ ID NOs:1-15.

Embodiment 4 is the recombinant DNA construct of embodiment 1, 2, or 3 wherein said DNA regulatory sequence has gene regulatory activity.

Embodiment 5 is the recombinant DNA construct of any one of embodiments 1-4, wherein said site-specific recombinase is selected from the group consisting of a Cre-recombinase, a Flp-recombinase, an R-recombinase, and a Gin-Recombinase.

Embodiment 6 is the recombinant DNA construct of any one of embodiments 1-5, wherein said site-specific recombinase is a Cre-recombinase.

Embodiment 7 is the recombinant DNA construct of any one of embodiments 1-6, further comprising one or both of the following expression cassettes: a selectable marker transgene; and/or a transgene of agronomic interest.

Embodiment 8 is the recombinant DNA construct of any one of embodiments 1-7, further comprising a pair of site-specific recombination site sequences flanking one or both of the transcribable DNA sequences encoding the site-specific recombinase and/or the selectable marker transgene, wherein the site-specific recombination sites can be cleaved by the site-specific recombinase.

Embodiment 9 is the recombinant DNA construct of embodiment 8, wherein said pair of site-specific recombination site sequences are oriented in a head-to-tail arrangement.

Embodiment 10 is the recombinant DNA construct of embodiment 8 or 9, wherein said selectable marker transgene confers resistance to a herbicide or an antibiotic.

Embodiment 11 is the recombinant DNA construct of any one of embodiments 8, 9 or 10, wherein said pair of site-specific recombination site sequences are each selected from the group consisting of LoxP, FRT, RS, and GIX.

Embodiment 12 is the recombinant DNA construct of any one of embodiments 7-11, or of any one of embodiments 8-11, wherein said pair of site-specific recombination site sequences are each a LoxP.

Embodiment 13 is the recombinant DNA construct of any one of embodiments 7-12, or of any one of embodiments 8-12, wherein said pair of site-specific recombination site sequences each comprise SEQ ID NO:20.

Embodiment 14 is the recombinant DNA construct of any one of embodiments 7-13, wherein said transgene of agronomic interest confers herbicide tolerance in plants.

Embodiment 15 is the recombinant DNA construct of any one of embodiments 7-13, wherein said transgene of agronomic interest confers pest or disease resistance in plants.

Embodiment 16 is the recombinant DNA construct of any one of embodiments 7-13, wherein said transgene of agronomic interest confers increased yield or stress tolerance in plants.

Embodiment 17 is the recombinant DNA construct of any one of embodiments 7-16, wherein said transgene of agronomic interest encodes a dsRNA, a miRNA, or an siRNA.

Embodiment 18 is the recombinant DNA construct of any one of embodiments 1-17, further comprising one or both of the following: an expression cassette encoding a guide RNA; and/or an expression cassette encoding a site-specific nuclease.

Embodiment 19 is the recombinant DNA construct of embodiment 18, further comprising a pair of site-specific recombination site sequences flanking one or more of the transcribable DNA sequence encoding the site-specific recombinase, the selectable marker transgene, the expression cassette encoding the guide RNA, and/or the expression cassette encoding the site-specific nuclease, wherein the site-specific recombination sites can be cleaved by the site-specific recombinase.

Embodiment 20 is the recombinant DNA construct of embodiment 18 or 19, wherein said guide RNA comprises a targeting sequence that targets a sequence in the genome of a eukaryotic cell for genome editing or site-specific integration.

Embodiment 21 is the recombinant DNA construct of embodiment 20, wherein said eukaryotic cell is a plant cell.

Embodiment 22 is the recombinant DNA construct of any one of embodiments 18-21, comprising two or more expression cassettes encoding two or more guide RNAs.

Embodiment 23 is the recombinant DNA construct of any one of embodiments 18-22, wherein there are two, three, four, five, six, seven, eight, nine, or ten different expression cassettes encoding guide RNAs.

Embodiment 24 is the recombinant DNA construct of any one of embodiments 18-23, wherein said site-specific nuclease is a RNA-guided endonuclease.

Embodiment 25 is the recombinant DNA construct of embodiment 18-24, wherein said RNA-guided endonuclease is selected from the group consisting of Cas1, Cas1B, Cas2, Cas3, Cas4, Cas5, Cas6, Cas7, Cas8, Cas9, Cas10, Cas12a, Cys1, Cys2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, CasX, and CasY.

Embodiment 26 is the recombinant DNA construct of embodiment 25, wherein said RNA-guided endonuclease is Cas9 or Cas12a.

Embodiment 27 is a DNA molecule or vector comprising said recombinant DNA construct of any one of embodiments 1-26.

Embodiment 28 is a DNA transformation vector comprising the recombinant DNA construct of any one of embodiments 1-26 and a T-DNA segment bounded by a left border and right border.

Embodiment 29 is the DNA transformation vector of embodiment 28, wherein the transcribable DNA sequence encoding the site-specific recombinase is located between the left border and the right border of the T-DNA segment.

Embodiment 30 is a DNA transformation vector comprising the recombinant DNA construct of any one of embodiments 7-26 and a T-DNA segment with a left border and a right border, wherein one or more of the transcribable DNA sequence encoding the site-specific recombinase, the selectable marker transgene and/or the transgene of agronomic interest is/are located between the left border and the right border of the T-DNA segment.

Embodiment 31 is a DNA transformation vector comprising the recombinant DNA construct of any one of embodiments 18-26 and a T-DNA segment with a left border and a right border, wherein one or more of the transcribable DNA sequence encoding the site-specific recombinase, the selectable marker transgene, the transgene of agronomic interest, the expression cassette encoding the guide RNA and/or the expression cassette encoding the site-specific nuclease is/are located between the left border and the right border of the T-DNA segment.

Embodiment 32 is a transgenic plant, plant part or plant cell comprising the recombinant DNA construct of any one of embodiments 1-26.

Embodiment 33 is the transgenic plant, plant part or plant cell of embodiment 32, wherein said recombinant DNA construct is stably transformed into the genome of said transgenic plant, plant part or plant cell.

Embodiment 34 is the transgenic plant, plant part or plant cell of embodiment 32 or 33, wherein said transgenic plant, plant part or plant cell is a corn, soybean, cotton or canola plant, plant part or plant cell.

Embodiment 35 is a bacterial cell comprising the recombinant DNA construct of any one of embodiments 1-26, the DNA molecule or vector of embodiment 27, or the transformation vector of any one of embodiments 28-31.

Embodiment 36 is a method for producing a transgenic plant or plant part, comprising:
a. transforming a plant cell of an explant with a DNA molecule or vector comprising the recombinant DNA construct of any one of embodiments 1-25 or any one of embodiments 1-26 to produce one or more transformed plant cells comprising the recombinant DNA construct stably transformed into the genome of the one or more transformed plant cells;
b. regenerating or developing a transgenic plant from the explant, wherein the transgenic plant comprises the recombinant DNA construct stably transformed into the genome of one or more cells of the transgenic plant.

Embodiment 37 is the method of embodiment 36, wherein said plant cell is transformed via *Agrobacterium*-mediated transformation or *Rhizobium*-mediated transformation.

Embodiment 38 is the method of embodiment 36, wherein said plant cell is transformed via microprojectile-mediated transformation or particle bombardment-mediated transformation.

Embodiment 39 is the method of embodiment 36, wherein said transgenic plant and plant cell are a corn, soybean, cotton or canola plant and plant cell, respectively.

Embodiment 40 is the method of embodiment 36, further comprising:
c. separating or harvesting a plant part from the transgenic plant.

Embodiment 41 is a method for excising an expression cassette from the genome of a transgenic plant, comprising:
a. transforming a plant cell with a DNA molecule or vector comprising the recombinant DNA construct of any one of embodiments 8-24 to produce one or more transformed plant cells comprising the recombinant DNA construct stably transformed into the genome of the one or more transformed plant cells;
b. regenerating or developing a transgenic plant at least in part from the one or more stably transformed plant cells;
c. crossing the transgenic plant to itself or another plant; and
d. selecting one or more progeny plants in which one or both of the transcribable DNA sequence encoding the site-specific recombinase and/or the selectable marker transgene between the pair of site-specific recombination site sequences of the recombinant DNA construct are excised and no longer present in the genome of the progeny plants.

Embodiment 42 is the method of embodiment 41,
wherein said recombinant DNA construct further comprises one or both of the following expression cassettes between the pair of DNA site-specific recombination site sequences of the recombinant DNA construct: an expression cassette encoding a guide RNA and/or an expression cassette encoding a site-specific nuclease, and
wherein one or more progeny plants are selected in which one or more of the transcribable DNA sequence encoding the site-specific recombinase, the selectable marker transgene, the expression cassette encoding the guide RNA, and/or the expression cassette encoding the site-specific nuclease of said recombinant DNA construct are excised and no longer present in the genome of the progeny plants.

Embodiment 43 is the method of embodiment 41, wherein said transgenic plant and plant cell are a corn, soybean, cotton or canola plant and plant cell, respectively.

Embodiment 44 is the method of embodiment 41, further comprising:
e. separating or harvesting a plant part from one or more of the progeny plants.

Embodiment 45 is the method of embodiment 41, further comprising:
f. crossing one or more of the progeny plants to itself or another plant.

Embodiment 46 is a recombinant DNA construct comprising a DNA sequence selected from the group consisting of:
d. a sequence with at least 85 percent identity to any of SEQ ID NOs:8, 10, 11, 12, and 14;
e. a sequence comprising any of SEQ ID NOs:8, 10, 11, 12, and 14; and
f. a fragment of any of SEQ ID NOs:8, 10, 11, 12, and 14, wherein the fragment has gene-regulatory activity;
wherein said sequence is operably linked to a heterologous transcribable DNA molecule.

Embodiment 47 is the recombinant DNA construct of embodiment 46, wherein said sequence has at least 90 percent sequence identity to the DNA sequence of SEQ ID NOs:8, 10, 11, 12, and 14.

Embodiment 48 is the recombinant DNA construct of embodiment 46 or 47, wherein said sequence has at least 95 percent sequence identity to the DNA sequence of SEQ ID NOs:8, 10, 11, 12, and 14.

Embodiment 49 is the recombinant DNA construct of embodiment 46, 47, or 48, wherein the DNA sequence comprises gene regulatory activity.

Embodiment 50 is the recombinant DNA construct of embodiment 46, wherein the heterologous transcribable DNA molecule comprises a gene of agronomic interest.

Embodiment 51 is the recombinant DNA construct of embodiment 50, wherein the gene of agronomic interest confers herbicide tolerance in plants.

Embodiment 52 is the recombinant DNA construct of embodiment 50, wherein the gene of agronomic interest confers pest resistance in plants.

Embodiment 53 is the recombinant DNA construct of embodiment 46, wherein the heterologous transcribable DNA molecule encodes a dsRNA, an miRNA, or a siRNA.

Embodiment 54 is a transgenic plant cell comprising a recombinant DNA construct comprising a sequence selected from the group consisting of:
g. a sequence with at least 85 percent sequence identity to any of SEQ ID NOs:8, 10, 11, 12, and 14;
h. a sequence comprising any of SEQ ID NOs:8, 10, 11, 12, and 14; and
i. a fragment of any of SEQ ID NOs:8, 10, 11, 12, and 14, wherein the fragment has gene-regulatory activity;
wherein said sequence is operably linked to a heterologous transcribable DNA molecule.

Embodiment 55 is the transgenic plant cell of embodiment 54, wherein said transgenic plant cell is a monocotyledonous plant cell.

Embodiment 56 is the transgenic plant cell of embodiment 54, wherein said transgenic plant cell is a dicotyledonous plant cell.

Embodiment 57 is a transgenic plant, or part thereof, comprising the recombinant DNA construct of embodiment 46.

Embodiment 58 is a progeny plant of the transgenic plant of embodiment 57, or a part thereof, wherein the progeny plant or part thereof comprises said recombinant DNA construct.

Embodiment 59 is a transgenic seed, wherein in the seed comprises the recombinant DNA construct of embodiment 46.

Embodiment 60 is a method of producing a commodity product comprising obtaining a transgenic plant or part thereof according to embodiment 57.

Embodiment 61 is the method of embodiment 60, wherein the commodity product is seeds, processed seeds, protein concentrate, protein isolate, starch, grains, plant parts, seed oil, biomass, flour, and meal.

Embodiment 62 is a method of expressing a transcribable DNA construct comprising obtaining a transgenic plant according to embodiment 57 and cultivating plant, wherein the transcribable DNA is expressed.

Embodiment 63 is a Cre-recombinase coding sequence with at least 90% sequence identity to SEQ ID NO:17.

Embodiment 64 is the Cre-recombinase coding sequence of embodiment 63 with at least 95% sequence identity to SEQ ID NO:17.

Embodiment 65 is the Cre-recombinase coding sequence of embodiment 63 or 64 with at least 99% sequence identity to SEQ ID NO:17.

Embodiment 66 is the method of any one of embodiments 36-38, wherein said transgenic plant and plant cell are a corn, soybean, cotton or canola plant and plant cell, respectively.

Embodiment 67 is the method of any one of embodiments 36-39, further comprising:
d. separating or harvesting a plant part from the transgenic plant.

Embodiment 68 is the method of embodiment 41 or 42, wherein said transgenic plant and plant cell are a corn, soybean, cotton or canola plant and plant cell, respectively.

Embodiment 69 is the method of any one of embodiments 41-43, further comprising:
f. separating or harvesting a plant part from one or more of the progeny plants.

Embodiment 70 is the method of any one of embodiments 41-44, further comprising:
g. crossing one or more of the progeny plants to itself or another plant.

Embodiment 71 is a transgenic plant, or part thereof, comprising the recombinant DNA construct of any one of embodiments 46-49.

Embodiment 72 is a progeny plant of the transgenic plant of embodiment 57 or 71, or a part thereof, wherein the progeny plant or part thereof comprises the recombinant DNA construct of any one of embodiments 46-49.

Embodiment 73 is a recombinant DNA construct comprising a DNA sequence selected from the group consisting of:
j. a sequence with at least 85 percent identity to any of SEQ ID NOs:1-15;
k. a sequence comprising any of SEQ ID NOs:1-15; and
l. a fragment of any of SEQ ID NOs: 1-15, wherein the fragment has gene-regulatory activity;
wherein said sequence is operably linked to a heterologous transcribable DNA molecule.

Embodiment 74 is the recombinant DNA construct of embodiment 73, wherein said sequence has at least 90 percent sequence identity to the DNA sequence of SEQ ID NOs:1-15.

Embodiment 75 is the recombinant DNA construct of embodiment 73 or 74, wherein said sequence has at least 95 percent sequence identity to the DNA sequence of SEQ ID NOs:1-15.

Embodiment 76 is the recombinant DNA construct of any one of embodiments 73-75, wherein the DNA sequence comprises gene regulatory activity.

Embodiment A1 is a recombinant DNA construct comprising a DNA regulatory sequence comprising:
a. a sequence with at least 80% sequence identity to any of SEQ ID NOs:1-15;
b. a sequence comprising any of SEQ ID NOs:1-15; and
c. a fragment of (i) any of SEQ ID NOs:1-15 or (ii) any sequence with at least 80% sequence identity to any of SEQ ID NOs:1-15, wherein the fragment has gene regulatory activity;
wherein said DNA regulatory sequence is operably linked to a heterologous transcribable DNA sequence encoding a site-specific recombinase.

Embodiment A2 is the recombinant DNA construct of embodiment A1 , wherein:
a. said DNA regulatory sequence has at least 90 percent sequence identity to the DNA sequence of any of SEQ ID NOs:1-15;
b. said DNA regulatory sequence has at least 95 percent sequence identity to the DNA sequence of any of SEQ ID NOs:1-15;
c. said DNA regulatory sequence has gene regulatory activity;
d. said site-specific recombinase is selected from the group consisting of a Cre-recombinase, a Flp-recombinase, an R-recombinase, and a Gin-Recombinase;
e. said site-specific recombinase is a Cre-recombinase;
f. the recombinant DNA construct further comprises one or both of the following expression cassettes: a selectable marker transgene; and/or a transgene of agronomic interest;
g. the recombinant DNA construct further comprises a pair of site-specific recombination site sequences flanking one or both of the transcribable DNA sequences encoding the site-specific recombinase and/or the selectable marker transgene, wherein the site-specific recombination sites can be cleaved by the site-specific recombinase; or
h. the recombinant DNA construct further comprises one or both of the following: an expression cassette encoding a guide RNA; and/or an expression cassette encoding a site-specific nuclease.

Embodiment A3 is the recombinant DNA construct of embodiment A2, wherein:
a. said pair of site-specific recombination site sequences are oriented in a head-to-tail arrangement;
b. said selectable marker transgene confers resistance to an herbicide or antibiotic;
c. said pair of site-specific recombination site sequences are each selected from the group consisting of LoxP, FRT, RS, and GIX;
d. said pair of site-specific recombination site sequences are each a LoxP;
e. said pair of site-specific recombination site sequences each comprise SEQ ID NO:20;
f. said transgene of agronomic interest confers herbicide tolerance in plants;
g. said transgene of agronomic interest confers pest or disease resistance in plants;
h. said transgene of agronomic interest confers increased yield or stress tolerance in plants;
i. said transgene of agronomic interest encodes a dsRNA, a miRNA, or an siRNA;
j. the recombinant DNA construct further comprises a pair of site-specific recombination site sequences flanking one or more of the transcribable DNA sequence encoding the site-specific recombinase, the selectable marker transgene, the expression cassette encoding the guide RNA, and/or the expression cassette encoding the site-specific nuclease, wherein the site-specific recombination sites can be cleaved by the site-specific recombinase;
k. said guide RNA comprises a targeting sequence that targets a sequence in the genome of a eukaryotic cell for genome editing or site-specific integration;
l. the recombinant DNA construct comprises two or more expression cassettes encoding two or more guide RNAs;
m. the recombinant DNA construct comprises two, three, four, five, six, seven, eight, nine, or ten different expression cassettes encoding guide RNAs;
n. said site-specific nuclease is a RNA-guided endonuclease; or
o. said RNA-guided endonuclease is selected from the group consisting of Cas1, Cas1B, Cas2, Cas3, Cas4, Cas5, Cas6, Cas7, Cas8, Cas9, Cas10, Cas12a, Cys1, Cys2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, CasX, and CasY.

Embodiment A4 is the recombinant DNA construct of embodiment A3, wherein:
a. said eukaryotic cell is a plant cell; or
b. said RNA-guided endonuclease is Cas9 or Cas12a.

Embodiment A5 is a DNA molecule, DNA vector, or DNA transformation vector comprising:
a. said recombinant DNA construct of embodiment A1; or
b. said recombinant DNA construct embodiment A1 and a T-DNA segment bounded by a left border and right border.

Embodiment A6 is the DNA transformation vector of embodiment A5, wherein said transcribable DNA sequence encoding the site-specific recombinase is located between the left border and the right border of the T-DNA segment.

Embodiment A7 is a DNA transformation vector comprising the recombinant DNA construct of embodiment A2, and a T-DNA segment with a left border and a right border, wherein:
a. one or more of the transcribable DNA sequence encoding the site-specific recombinase, the selectable marker transgene and/or the transgene of agronomic interest is/are located between the left border and the right border of the T-DNA segment; or
b. one or more of the transcribable DNA sequence encoding the site-specific recombinase, the selectable marker transgene, the transgene of agronomic interest, the expression cassette encoding the guide RNA and/or the expression cassette encoding the site-specific nuclease is/are located between the left border and the right border of the T-DNA segment.

Embodiment A8 is a transgenic plant, plant part or plant cell, or a bacterial cell comprising said recombinant DNA construct of embodiment A1.

Embodiment A9 is the transgenic plant, plant part or plant cell of embodiment A8, wherein:
a. said recombinant DNA construct is stably transformed into the genome of the transgenic plant, plant part or plant cell; or
b. said transgenic plant, plant part or plant cell is a corn, soybean, cotton or canola plant, plant part or plant cell.

Embodiment A10 is a method for producing a transgenic plant or plant part, comprising:
a. transforming a plant cell of an explant with a DNA molecule or vector comprising the recombinant DNA construct of embodiment A1 to produce one or more transformed plant cells comprising the recombinant DNA construct stably transformed into the genome of the one or more transformed plant cells; and
b. regenerating or developing a transgenic plant from the explant, wherein the transgenic plant comprises the recombinant DNA construct stably transformed into the genome of one or more cells of the transgenic plant.

Embodiment A11 is the method of embodiment A10, wherein:
a. said plant cell is transformed via *Agrobacterium-*mediated transformation or *Rhizobium-*mediated transformation;
b. said plant cell is transformed via microprojectile-mediated transformation or particle bombardment-mediated transformation;
c. said transgenic plant and plant cell are a corn, soybean, cotton or canola plant and plant cell, respectively; or
d. the method further comprises: separating or harvesting a plant part from the transgenic plant.

Embodiment A12 is a method for excising an expression cassette from the genome of a transgenic plant, comprising:
a. transforming a plant cell with a DNA molecule or vector comprising the recombinant DNA construct of embodiment A2 to produce one or more transformed plant cells comprising the recombinant DNA construct stably transformed into the genome of the one or more transformed plant cells;
b. regenerating or developing a transgenic plant at least in part from the one or more stably transformed plant cells;
c. crossing the transgenic plant to itself or another plant; and
d. selecting one or more progeny plants in which one or both of the transcribable DNA sequence encoding the site-specific recombinase and/or the selectable marker transgene between the pair of site-specific recombination site sequences of the recombinant DNA construct are excised and no longer present in the genome of the progeny plants.

Embodiment A13 is the method of embodiment A12, wherein:
a. said recombinant DNA construct further comprises one or both of the following expression cassettes between the pair of DNA site-specific recombination site sequences of the recombinant DNA construct: an expression cassette encoding a guide RNA and/or an expression cassette encoding a site-specific nuclease, and wherein one or more progeny plants are selected in which one or more of the transcribable DNA sequence encoding the site-specific recombinase, the selectable marker transgene, the expression cassette encoding the guide RNA, and/or the expression cassette encoding the site-specific nuclease of said recombinant DNA construct are excised and no longer present in the genome of the progeny plants;
b. said transgenic plant and plant cell are a corn, soybean, cotton or canola plant and plant cell, respectively;
c. the method further comprises: separating or harvesting a plant part from one or more of the progeny plants; or
d. the method further comprises: crossing one or more of the progeny plants to itself or another plant.

Embodiment A14 is a recombinant DNA construct comprising a DNA sequence selected from the group consisting of:
a. a sequence with at least 85 percent identity to any of SEQ ID NOs:8, 10, 11, 12, and 14;
b. a sequence comprising any of SEQ ID NOs:8, 10, 11, 12, and 14; and
c. a fragment of any of SEQ ID NOs:8, 10, 11, 12, and 14, wherein the fragment has gene-regulatory activity;
wherein said sequence is operably linked to a heterologous transcribable DNA molecule.

Embodiment A15 is the recombinant DNA construct of embodiment A14, wherein:
a. said sequence has at least 90 percent sequence identity to the DNA sequence of SEQ ID NOs:8, 10, 11, 12, and 14;
b. said sequence has at least 95 percent sequence identity to the DNA sequence of SEQ ID NOs:8, 10, 11, 12, and 14;
c. the DNA sequence comprises gene regulatory activity;
d. the heterologous transcribable DNA molecule comprises a gene of agronomic interest; or
e. the heterologous transcribable DNA molecule encodes a dsRNA, an miRNA, or a siRNA.

Embodiment A16 is the recombinant DNA construct of embodiment A15, wherein:
a. the gene of agronomic interest confers herbicide tolerance in plants; or
b. the gene of agronomic interest confers pest resistance in plants.

Embodiment A17 is a transgenic plant cell comprising a recombinant DNA construct comprising a sequence selected from the group consisting of:
a. a sequence with at least 85 percent sequence identity to any of SEQ ID NOs:8, 10, 11, 12, and 14;
b. a sequence comprising any of SEQ ID NOs:8, 10, 11, 12, and 14; and
c. a fragment of any of SEQ ID NOs:8, 10, 11, 12, and 14, wherein the fragment has gene-regulatory activity;
wherein said sequence is operably linked to a heterologous transcribable DNA molecule.

Embodiment A18 is the transgenic plant cell of embodiment A17, wherein:
a. said transgenic plant cell is a monocotyledonous plant cell; or
b. said transgenic plant cell is a dicotyledonous plant cell.

Embodiment A19 is a transgenic plant or plant part, transgenic plant seed, or progeny plant or plant part thereof, comprising the recombinant DNA construct of embodiment A14.

Embodiment A20 is a method of producing a commodity product comprising obtaining a transgenic plant or part thereof according to embodiment A19.

Embodiment A21 is the method of embodiment A20, wherein the commodity product is seeds, processed seeds, protein concentrate, protein isolate, starch, grains, plant parts, seed oil, biomass, flour, and meal.

Embodiment A22 is a method of expressing a transcribable DNA construct comprising obtaining a transgenic plant according to embodiment A19 and cultivating said plant, wherein the transcribable DNA is expressed.

Embodiment A23 is a Cre-recombinase coding sequence, said sequence having:
a. at least 90% sequence identity to SEQ ID NO:17.
b. at least 95% sequence identity to SEQ ID NO:17; or
c. at least 99% sequence identity to SEQ ID NO:17.

Having illustrated and described the principles of the present invention, it should be apparent to persons skilled in the art that the invention can be modified in arrangement and detail without departing from such principles. We claim all modifications that are within the spirit and scope of the claims. All publications and published patent documents cited herein are hereby incorporated by reference to the same extent as if each individual publication or patent application is specifically and individually indicated to be incorporated by reference.

## Claims

1. A recombinant DNA construct comprising a DNA sequence selected from the group consisting of:
a. a sequence with at least 85 percent sequence identity to SEQ ID NO:10 or SEQ ID NO:11;
b. a sequence comprising SEQ ID NO:10 or SEQ ID NO:11; and
c. a fragment of SEQ ID NO:10 or SEQ ID NO:11, wherein the fragment has gene-regulatory activity;
wherein said sequence is operably linked to a heterologous transcribable DNA molecule.

2. The recombinant DNA construct of claim 1, wherein the DNA sequence has at least 90 percent sequence identity to the DNA sequence of SEQ ID NO:10 or SEQ ID NO:11.

3. The recombinant DNA construct of claim 1, wherein the DNA sequence has at least 95 percent sequence identity to the DNA sequence of SEQ ID NO:10 or SEQ ID NO:11.

4. The recombinant DNA construct of claim 1, wherein the DNA sequence has gene-regulatory activity.

5. The recombinant DNA construct of claim 1, wherein the heterologous transcribable DNA molecule comprises a gene of agronomic interest.

6. The recombinant DNA construct of claim 5, wherein the gene of agronomic interest confers herbicide tolerance in plants.

7. The recombinant DNA construct of claim 5, wherein the gene of agronomic interest confers pest resistance in plants.

8. The recombinant DNA construct of claim 1, wherein the heterologous transcribable DNA molecule encodes a dsRNA, a miRNA, or an siRNA.

9. The recombinant DNA construct of claim 1, wherein the heterologous transcribable DNA molecule encodes a site-specific recombinase.

10. A transgenic plant cell comprising a recombinant DNA construct comprising a DNA sequence selected from the group consisting of:
d. a sequence with at least 85 percent sequence identity to SEQ ID NO:10 or SEQ ID NO:11;
e. a sequence comprising SEQ ID NO:10 or SEQ ID NO:11; and
f. a fragment of SEQ ID NO:10 or SEQ ID NO:11, wherein the fragment has gene-regulatory activity;
wherein said sequence is operably linked to a heterologous transcribable DNA molecule.

11. The transgenic plant cell of claim 10, wherein:
c. said transgenic plant cell is a monocotyledonous plant cell; or
d. said transgenic plant cell is a dicotyledonous plant cell.

12. A transgenic plant or plant part, or a transgenic plant seed, comprising the recombinant DNA construct of claim 1.

13. A method of producing a commodity product comprising the step of obtaining a transgenic plant or part thereof according to claim 12.

14. The method of claim 13, wherein the commodity product is selected from the group consisting of seeds, processed seeds, protein concentrate, protein isolate, starch, grains, plant parts, seed oil, biomass, flour, and meal.

15. A method of expressing a transcribable DNA molecule comprising the step of obtaining a transgenic plant according to claim 12 and cultivating said plant, wherein the transcribable DNA molecule is expressed.
